# EUROPEAN PATENT SPECIFICATION

(11) **EP 4 484 638 B1**
(45) Date of publication and mention of the grant of the patent: **10.06.2026**
(21) Application number: 23760267.7
(22) Date of filing: 27.01.2023
(51) Int. Cl.: D06F 58/38, D06F 58/20, D06F 58/26, D06F 39/00, D06F 34/18, A61L 2/07, D06F 73/02, D06F 39/40

(54) **CLOTHING TREATMENT APPARATUS**
KLEIDUNGSBEHANDLUNGSVORRICHTUNG
APPAREIL DE TRAITEMENT DE VÊTEMENTS

(30) Priority: 25.02.2022 KR 20220025494
(43) Date of publication of application: 01.01.2025
(73) Proprietor: LG Electronics Inc., Yeongdeungpo-gu Seoul 07336 (KR)
(72) Inventor: JANG, Jinhyuk, Seoul 08592 (KR); KIM, Minji, Seoul 08592 (KR); KIM, Jaehyung, Seoul 08592 (KR); PARK, Sunghoo, Seoul 08592 (KR)
(74) Representative: Vossius & Partner Patentanwälte Rechtsanwälte mbB
(86) International application number: PCT/KR2023/001286
(87) International publication number: WO 2023/163393

(56) References cited:
- EP-A1- 3 868 943
- JP-A- 2013 090 815
- KR-A- 20070 121 970
- KR-A- 20180 037 459
- KR-A- 20200 057 545
- KR-A- 20210 056 307
- KR-B1- 100 811 662
- US-A1- 2001 037 590

## Description

### TECHNICAL FIELD

The present invention relates to a clothing treatment apparatus, more particularly, to a clothing treatment apparatus for performing a refresh cycle such as sterilizing, removing wrinkles, deodorizing, and drying of laundry by supplying steam and hot air to the laundry.

### BACKGROUND

In general, a clothing treatment apparatus is a concept including a washing machine that soaks laundry in water to be wet and then removes foreign substances through a chemical action of detergent and a physical action such as drum rotation, and a dryer that dries the wet laundry by using hot air and steam.

However, recently, laundry care machines that keep dried laundry comfortable and clean without soaking the laundry in water have appeared. Such a laundry care machine may perform a refresh cycle of deodorizing the laundry and drying or sterilizing the laundry by supplying steam or hot air while the laundry is placed.

The laundry care machine may selectively add fragrance to the laundry, and in recent years, have become an important part of clothing treatment apparatuses along with washing machines and dryers.

The laundry care machine that performs a refreshing cycle for the laundry accommodates dry laundry, and thus a steam supply portion that supplies steam to the laundry is required.

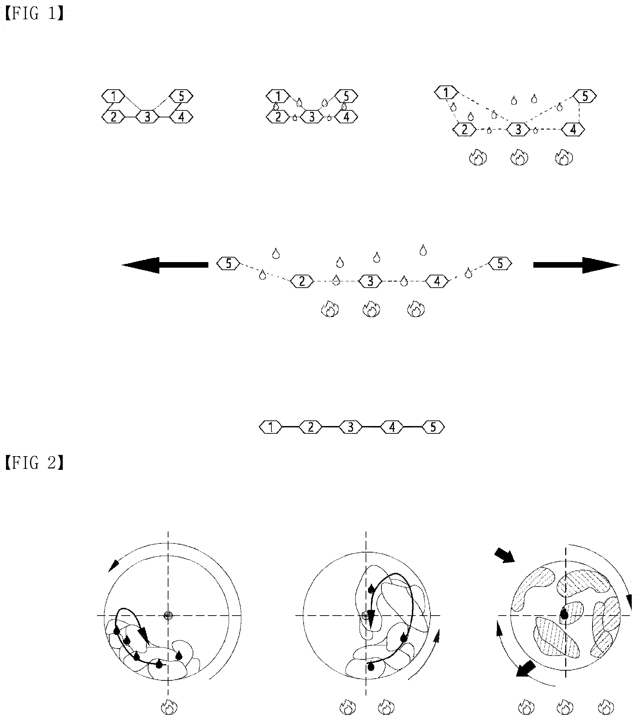

FIG. 1 shows a conventional clothing treatment apparatus including a steam supply portion.

Referring to Korean Patent Publication No. 10-2020-0057545, the conventional clothing treatment apparatus includes a cabinet 1 defining an outer appearance, and an inner case 2 provided inside the cabinet 1 to hold laundry.

The clothing treatment apparatus may include a circulation duct 13 located at a lower portion of the inner case 2 and circulating air of the inner case 2, a heat exchanger 15 provided in the circulation duct 13 to exchange heat with the air, and a compressor 14 that supplies high-temperature refrigerant to the heat exchanger 15. When the compressor 14 is driven, hot air is supplied to the inner case 2 to increase the temperature inside the inner case 2, and the laundry may be dried or sterilized.

The clothing treatment apparatus may include a steam tank 16 provided outside the circulation duct 13 to store water, and a heater 17 accommodated in the steam tank 16 to heat water to generate steam. When the heater 17 is driven, steam is supplied inside the inner case 2 to deodorize the laundry or remove wrinkles from the laundry.

The laundry accommodated inside the inner case 2 are placed in a dry state. Therefore, for the laundry to be effectively refreshed, a sufficient amount of moisture needs to be supplied to the inner case 2 to refresh the laundry.

Accordingly, the heater 17 may be provided with a relatively large capacity such that the laundry placed in the inner case 2 may absorb a sufficient amount of moisture. For example, the heater 17 may have a larger electric capacity than a heater applied to a washing machine or dryer that supplies steam to wet laundry.

As a result, the conventional clothing treatment apparatus has a fundamental limitation in that the heater 17 and the compressor 14 may not be driven simultaneously not to exceed an allowable electric capacity.

Therefore, when the heater 17 is driven to supply steam into the inner case 2, there is a problem in which hot air is not supplied into the inner case 2. In other words, the moisture content of the laundry may be increased by supplying steam and the temperature inside the inner case 2 may be raised to a certain level, but there is a problem in which the temperature inside the inner case 2 is not maintained above the minimum required for deodorization, sterilization, and drying.

As a result, when the steam 2 is supplied inside the inner case 2, there is a limitation in ensuring the deodorizing and sterilizing performance of the laundry.

The conventional clothing treatment apparatus requires a sufficient amount of moisture supply rather than hot air to refresh the laundry, and all of the supplied moisture needs to be dried, and thus the compressor 17 needs to be operated after driving of the heater 17 is completed.

As a result, in the conventional clothing treatment apparatus, the temperature inside the inner case 2 may not be maintained at the minimum temperature until the compressor 17 is driven, and thus there is a problem in that an effect of the refresh cycle is not obtained.

Therefore, the temperature inside the inner case 2 may be raised and maintained at the minimum temperature only at a subsequent point when the compressor 17 is driven in the refresh cycle, and thus there is a fundamental limitation that the cycle of refreshing the laundry itself is inevitably delayed significantly.

In addition, at an initial point when the compressor 17 is driven, steam may not be supplied into the inner case 2, and thus the air in contact with the heat exchanger 15 is at a relatively low temperature. As a result, air discharged from the inner case 2 does not ensure enough heat to exchange heat with the refrigerant flowing through the heat exchanger 15, and thus there is a problem in that a coefficient of performance (COP) of the heat pump system is not be ensured.

As described above, the heater 6 is provided with a large capacity to increase the moisture content of laundry inside the inner case 2, and thus when the heater 6 operates, the humidity inside the inner case 2 rapidly increase.

Therefore, the conventional clothing treatment apparatus has a problem in that the apparatus may not manage laundry that is vulnerable to moisture, such as silk or cashmere.

A clothing treatment apparatus including a steam supply portion that supplies steam to a drum containing laundry from among the washing machine or the dryer has also appeared.

FIG. 2 shows a conventional clothing treatment apparatus including a steam supply portion for drying.

Referring to Korean Patent No. 10-1448632, the conventional clothing treatment apparatus may include a laundry receiver 2 that accommodates laundry inside a cabinet 1, a water supply pipe 5 that supplies water into the laundry receiver 2, and a steam supply portion 4 connected to the water supply pipe 5 to receive water and supply steam to the laundry receiver 2.

The steam supply portion 4 may include a heater 6 inside that heats water to generate steam. Accordingly, the conventional clothing treatment apparatus sprays steam into the laundry receiver 2 through the steam supply portion 4 during a washing or drying cycle, and thus the temperature inside the laundry receiver 2 may be increased to increase washing efficiency or drying efficiency, remove wrinkles from the laundry, or perform sterilization.

However, the conventional clothing treatment apparatus such as the washing machine or dryer are assumed to process wet laundry, and thus there is no need to generate a large amount of steam to increase the moisture content of dry laundry, such as a laundry care machine inside the laundry receiver 2.

Therefore, the conventional clothing treatment apparatus shown in FIG. 2 does not need to include a large-capacity heater 6 like a laundry care machine.

In the conventional clothing treatment apparatus, the heater 6 may also include a first heater 61 and a second heater 62 to be driven independently of the first heater 61 to adjust a steam supply portion amount.

However, the conventional clothing treatment apparatus such as a washing machine and a dryer performs a dehydration cycle or a drying cycle to remove moisture from wet laundry. Therefore, there is a fundamental limitation in that the heater 6 is not operated when a heat pump system including a compressor or a heating system (hereinafter a heat supply portion) including an air heating heater is driven while the laundry receiver 2 rotates or the drying cycle is performed.

As a result, even if dry laundry is put into the washing machine and the dryer, there is a fundamental limitation in that the heater 6 and the heat supply portion or a motor may not be driven simultaneously to refresh the laundry.

When a drying course is performed in a clothing treatment apparatus such as the washing machine and the dryer, the purpose is to final dry the wet laundry, and thus even if the heater 6 is heated and supplies steam during the drying course, when the heat supply portion starts operating, there is no stopping until completion. In this case, not only does the drying performance decrease drastically, but the drying course is also greatly delayed.

Accordingly, there is a limit that the washing machine and the dryer have no possibility or implication of not only a technology of simultaneously driving a heater and a compressor that generates steam, but also a technology of stopping driving of a compressor to drive the heater that generates steam after starting the operation of the compressor.

As a result, the conventional clothing treatment apparatus is a technology in which a heater that generates steam and a compressor that generates hot air operate simultaneously in the laundry care machine, but has a limitation in not providing technical details on how to control and apply a heater in the laundry care machine for processing dry laundry when the heater is provided as a dual heater.

The conventional clothing treatment apparatus had a problem in that it did not provide any standards for determining the specifications of the heater that generates steam or a control method for operating the heater and the compressor in conjunction.

KR 2020 0057545 A discloses a cabinet-type clothes treating apparatus using a heat pump for supplying hot air into a cabinet space and a steam generator for supply steam into the cabinet space.

### DISCLOSURE

### Technical Problem

An object of the present invention is to simultaneously drive a compressor and some heater from among a plurality of heaters generating steam and provide the standard for optimally determining the specifications of the plurality of heaters.

An object of the present invention is to provide a clothing treatment apparatus with specifications by which the temperature inside an inner case is raised to a target temperature while each heater uses water below an allowable value.

An object of the present invention is to provide a clothing treatment apparatus for optimally determining specifications of each heater based on a dehumidification amount of an evaporator that condenses moisture.

An object of the present invention is to provide a clothing treatment apparatus for driving a steam heater to ensure the reliability of a compressor.

### Technical Solution

The present invention defined in the appended claims solves the above-described problems. Specifically, the present invention optimally determines specifications of a first heater and a second heater that supply steam to the inside of the inner case in which laundry is held.

For example, the first heater may be provided such that the amount of steam generated by the first heater is greater than a dehumidification amount of an evaporator, and the second heater may be provided such that the amount of steam generated by the second heater is equal to or less the dehumidification amount of the evaporator.

The first heater may be provided such that an average steam generation amount per minute is greater than an average dehumidification amount per minute of the evaporator, and the second heater may be provided such that the average steam generation per minute is equal to or less than the average dehumidification amount per minute of the evaporator.

The first heater may be provided such that the maximum steam generation amount is greater than the maximum dehumidification amount of the evaporator, and the second heater may be provided such that the maximum steam generation amount is equal to or less than the maximum dehumidification amount of the evaporator.

When the compressor and the first heater are simultaneously driven for more than a certain time, the humidity inside the inner case may increase.

When the compressor and the second heater are simultaneously driven for more than a certain time, the humidity inside the inner case may be maintained or decreased.

When the compressor, the first heater, and the second heater are driven simultaneously for more than a certain time, the humidity inside the inner case may increase.

The average steam generation amount of the second heater may be set to be less than the average dehumidification amount of the evaporator.

The first heater or the second heater is provided such that the amount of steam generated in the basic course corresponds to 80 to 120% of the dehumidification amount of the evaporator.

The first heater or the second heater may be provided such that the amount of steam generated in the intensive course corresponds to 130 to 150% of the dehumidification amount of the evaporator.

The amount of steam generated by the second heater may be set to be less than that of the first heater.

The controller may intermittently and repeatedly drive either the first heater or the second heater when the compressor is driven during at least a portion of the course.

The heat supply portion may include a refrigerant sensor configured to detect the temperature of the refrigerant discharged from the compressor or the condenser.

The controller may intermittently and repeatedly drive the first heater or the second heater when the temperature of the refrigerant flowing into or discharged from the compressor reaches a limit temperature while the first heater or the second heater and the compressor are driven simultaneously.

The clothing treatment apparatus may further include a temperature sensor configured to detect the internal temperature of the inner case or the temperature of the air flowing into the circulation duct.

When the temperature reaches the target temperature while the first heater or the second heater and the compressor are simultaneously driven, the controller may intermittently and repeatedly drive the first heater or the second heater.

When the first heater or the second heater is intermittently driven, a driving time may be set to be longer than a time when driving is stopped.

The second heater, a steam generation amount of which is set to be less than that of the first heater, may be set to have a specification for raising the internal temperature of the inner case to at least the target temperature.

The second heater may be set to have a specification for raising the internal temperature of the inner case to at least the target temperature before the delay time elapses.

The second heater may be set to have a specification for increasing the internal temperature of the inner case to the target temperature while using water below an allowable value.

The driving power of the second heater may be set between 450 w and 700 w.

The driving power of the first heater may be set between 800 w to 1100 w.

### Advantageous Effects

The present invention has an effect of simultaneously driving a compressor and some heater from among a plurality of heaters generating steam and providing the standard for optimally determining the specifications of the plurality of heaters.

The present invention has an effect of obtaining specifications by which the temperature inside an inner case is raised to a target temperature while each heater uses water below an allowable value.

The present invention has an effect of optimally determining specifications of each heater based on a dehumidification amount of an evaporator that condenses moisture.

The present invention has an effect of driving a steam heater to ensure the reliability of a compressor.

### BRIEF DESCRIPTION OF THE DRAWINGS

FIG. 1 shows a condition for removing wrinkles and creases.
FIG. 2 shows whether a removal condition of a conventional dryer is achieved.
FIG. 3 shows an outer appearance of a clothing treatment apparatus according to the present invention.
FIG. 4 shows a machine room structure of a clothing treatment apparatus.
FIG. 5 shows a circulation duct structure of a clothing treatment apparatus.
FIG. 6 shows a heat supply portion of a clothing treatment apparatus.
FIG. 7 shows a blowing fan of a clothing treatment apparatus.
FIG. 8 shows an inlet duct structure of a clothing treatment apparatus.
FIG. 9 shows a steam supply portion of a clothing treatment apparatus.
FIG. 10 shows a steam heater of a clothing treatment apparatus.
FIG. 11 shows the inside of a steam case of a clothing treatment apparatus.
FIG. 12 shows an internal structure of a steam supply portion of a clothing treatment apparatus.
FIG. 13 shows a flow path structure of a clothing treatment apparatus.
FIG. 14 is a schematic diagram showing a flow path structure of a clothing treatment apparatus.
FIG. 15 shows a movement directions of steam and water in a clothing treatment apparatus.
FIG. 16 shows a moving hanger structure of a clothing treatment apparatus.
FIG. 17 shows an operation method of a moving hanger of a clothing treatment apparatus.
FIG. 18 shows moving hanger structures of a clothing treatment apparatus.
FIG. 19 shows a moving hanger structure of a clothing treatment apparatus.
FIG. 20 shows an operation structure of a moving hanger of a clothing treatment apparatus.
FIG. 21 shows a process in which a clothing treatment apparatus performs a laundry treating course.
FIG. 22 shows an embodiment in which a specification of a steam heater is determined based on temperature.
FIG. 23 show an embodiment in which a specification of a steam heater is based on temperature and water usage.
FIG. 24 shows an embodiment in which a specification of a steam heater is determined based on a dehumidification amount of a heat supply portion.
FIG. 25 shows an embodiment in which a specification of a steam heater is determined based on a dehumidification amount of a heat supply portion.
FIG. 26 shows an example of an optimal driving method of a steam heater.

### DETAILED DESCRIPTION

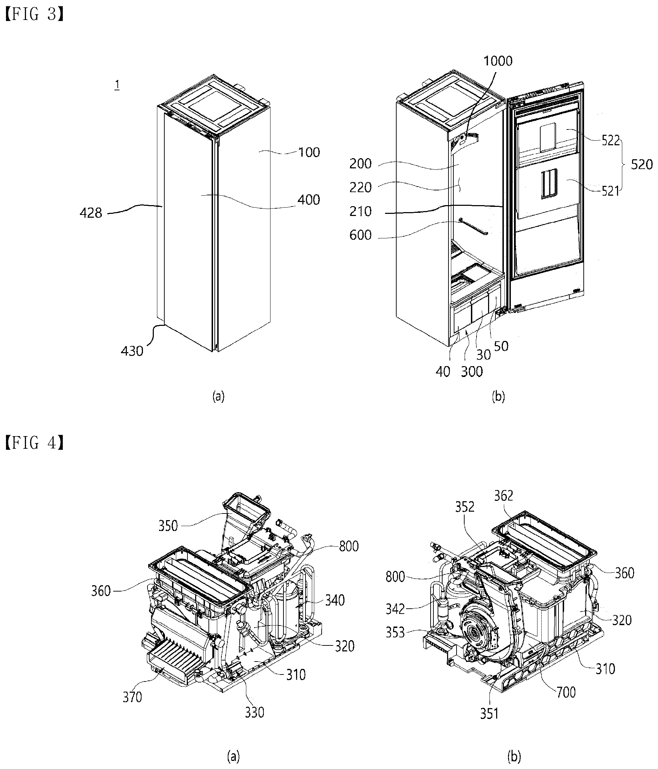

FIG. 3 shows an outer appearance of a clothing treatment apparatus 1 according to the present invention.

Referring to FIG. 3(a), the clothing treatment apparatus may include a cabinet 100 defining an outer appearance, and a door 400 rotatably coupled to the cabinet 100.

The door 400 may include a main body 410 that defines a front surface of the cabinet 100, and an installation body 420 that extends from one side of the main body 410 and on which a display displaying information about the clothing treatment apparatus is to be installed.

The installation body 420 may define a step 430 from the main body 410 toward a rear side of the cabinet 100.

At least a portion of the installation body 420 may be located to overlap a rear side of the main body 410 in front and rear directions. Accordingly, the step 430 may function as a handle.

The installation body 420 may include a different material or different color from the main body 410. The installation body 420 may include a translucent material through which light emitted from the display passes.

Referring to FIG. 3(b), an inner case 200 having a receiving space 220 for accommodating laundry may be provided inside the cabinet 100. The inner case 200 may include an opening 210 at a front side, for inserting and removing laundry, and the opening 210 may be shielded by the door 400.

The inner case 200 may include a plastic resin series, and may include a reinforced plastic resin series that is not deformed by air at a temperature higher than room temperature air, heated air (hereinafter referred to as hot air), steam, or moisture.

The inner case 200 may be provided with a height greater than a width. As a result, the laundry may be accommodated in the receiving space 220 without being folded or wrinkled.

The clothing treatment apparatus 1 may include a moving hanger 1000 for holding laundry in the receiving space 220 of the inner case 200.

The moving hanger 100 may be provided as a simple mounting portion mounted in an upper side of the inner case 200 to fix the laundry while floating inside the receiving space 220.

Alternatively, the moving hanger 1000 may be installed on an upper surface of the inner case 200 to shake the laundry.

The moving hanger 1000 may reciprocate or rotate reciprocally in the upper side of the inner case 200. As a result, laundry mounted on the moving hanger 100 may be shaken in the receiving space 220 to remove foreign substances and may be effectively exposed to supplied steam or hot air.

The detailed structure and function of the moving hanger 1000 will be described later.

A pressurization unit 520 for pressurizing laundry may be provided on an inner surface of the door 400.

The pressurization unit 520 may include a support 522 that is fixed to the inner surface of the door 400 and supports one surface of the laundry, and a pressurizer 521 that pressurizes the laundry supported on the support 522.

The pressurizer 521 may be moved toward the support 522 or may be moved away from the support 522. For example, the pressurizer 521 may be rotatably provided on the support 522 or the inner surface of the door 400.

Accordingly, the pressurizer 521 and the support 522 may press both surfaces of the laundry to remove wrinkles from the laundry and create intended creases.

The clothing treatment apparatus may include a machine room 300 in which various devices for supplying one or more of hot air or steam to the receiving space 220 or absorbing, purifying, or dehumidifying external air of the cabinet 100 and then discharging the air.

The machine room 300 may include a built-in controller for controlling the clothing treatment apparatus. The controller may also be installed inside the door 400.

The controller may be provided as a printed circuit board (PCB) that controls one or more of electrical components of the clothing treatment apparatus and performs an arbitrary course for processing the laundry.

The machine room 300 may be arranged separately or partitioned from the inner case 200 and may communicate with the inner case 200.

The machine room 300 may be located below the inner case 200. Accordingly, when hot air and steam with a small specific gravity are supplied to the inner case 200, the hot air and steam may be smoothly supplied to the laundry.

The machine room 300 may include a heat supply portion 340 for supplying hot air into the inner case 200. The heat supply portion 340 may be provided as a heat pump system or as a heater that directly heats air with electric energy.

When the heat supply portion 340 is provided as a heat pump system, the heat supply portion 340 may dehumidify and heat air discharged from the inner case 200 and supply the air to the inner case 200. The detailed structure is described later.

The machine room 300 may include a steam supply portion 800 for supplying steam into the inner case 200. The steam supply portion 800 may directly supply steam into the inner case 200. The detailed structure is described later.

To this end, the inner case 200 may include a plurality of through holes 230 that are formed to pass through one surface and communicate with the machine room 300.

Air in the receiving space 220 may be supplied to the machine room 300 through the through hole 230, and one or more of hot air or steam generated in the machine room 300 may be supplied to the receiving space 200.

The through hole 230 may include an inlet hole 231 that is formed to pass through a lower surface of the inner case 200 and introduce air inside the inner case 200 into the machine room 300, and an exhaust hole 232 that is formed to pass through the lower surface of the inner case 200 to be spaced apart from the inlet hole 231 and discharges hot air generated in the machine room 300 into the inner case 200.

The exhaust hole 232 may be located closer to a rear surface of the inner case 200 than a front surface of the lower surface the inner case 200 or the door 400.

The exhaust hole 232 may be located between the lower surface and the rear surface of the inner case 200 at an angle to the ground. The exhaust hole 232 may face the moving hanger 1000, and thus the hot air may be supplied toward the laundry.

The inlet hole 231 may be located closer to a front side or the door 400 than the rear surface of the lower surface of the inner case 200.

The inlet hole 231 may be located to be furthest from the exhaust hole 232 to prevent the air discharged from the exhaust hole 232from being absorbed directly into the inlet hole 231 without reaching the laundry.

The through hole 230 may further include a steam hole 233 that is formed to pass through a lower side of the inner case 200 and guides steam generated by the steam supply portion 800 into the inner case 200.

The steam hole 233 may be located closer to the exhaust hole 232 than the inlet hole 231. For example, the steam hole 233 may be located at one side of the exhaust hole 232.

The machine room 300 may further include a water supply tank 30 that supplies water to the steam supply portion 800, and a drain tank 40 in which condensed water condensed in the heat supply portion 340 is collected.

The water supply tank 30 and the drain tank 40 may be detachably provided from a front side of the machine room 300. Accordingly, the clothing treatment apparatus 1 may be freely installed without being restricted from a water supply source or a drainage source.

The machine room 300 may further include a drawer 50 that is retracted into and extended out of the front side and has a separate receiving space. For example, the drawer 50 may store a steam generator or an iron.

FIG. 4 shows a machine room structure of a clothing treatment apparatus.

FIG. 4(a) is a diagram of the machine room 300 from the front, and FIG. 4(b) is a diagram of the machine room 300 from the rear.

Components may be placed inside the machine room 300 to supply hot air to a laundry treating space, circulate air inside the laundry treating space, supply steam to the laundry treating space, or clean the air outside the cabinet.

The machine room 300 may include a base portion 310 that supports various devices or arranges a space for installation the devices. The base portion 310 may provide an area in which various devices are to be installed.

The base portion 310 may include a circulation duct 320 installed therein, through which air introduced from outside the inner case 200 or the cabinet 100 moves.

The circulation duct 320 may be provided in a case shape with an open top, and some components of the heat supply portion 340 may be installed therein.

When the heat supply portion 340 is provided as a heat pump system, the heat supply portion 340 may include heat exchangers 341 and 343, which will be described later, inside the circulation duct 320, and a compressor 342 that supplies high-temperature and high-pressure refrigerant to the heat exchanger.

The heat exchangers 341 and 343 may be accommodated inside the circulation duct 320 and may cool and dehumidify air flowing through the circulation duct 320 or heat the air to generate hot air.

When the circulation duct 320 is provided to suck air from the outside of the cabinet 100, an outside air duct 370 may be installed in front of the circulation duct 320 to suck in outside air.

The circulation duct 320 may be provided to communicate with the outside air duct 370 to selectively suck in outside air.

The water supply tank and the drain tank may be detachably coupled to a front surface of the circulation duct 320. The water supply tank 30 and the drain tank 40 may be accommodated and located above the outside air duct 370.

The circulation duct 320 may be coupled to the base portion 310 or may be provided integrally with the base portion 310. For example, the base portion 310 and the circulation duct 320 may be manufactured by injection molding.

The machine room 300 may include a base cover 360 provided to communicate with the circulation duct 320 and the inlet hole 231.

The base cover 360 may be coupled to an upper side of the circulation duct 320 to guide air sucked in from the inlet hole 231 into the circulation duct 320.

The base cover 360 may block the air inside the circulation duct 320 from being discharged to the outside by shielding the upper surface of the circulation duct 320. The lower side of the base cover 360 and the upper surface of the circulation duct 320 may form one surface of a flow path of the circulation duct 320.

The base cover 360 may include an inlet 362 connecting the inlet hole 231 and the circulation duct 320 to each other. The inlet 362 may be provided in a duct shape and may function as an intake duct that delivers air inside the inner case 200 to the circulation duct 320.

The machine room 300 may include the steam supply portion 800 installed therein, which is connected to the water supply tank 30, receives water, generates steam, and supplies the steam to the inner case 200. The steam supply portion 800 may be accommodated and located on the upper side the base cover 360.

The steam supply portion 800 may be located outside the circulation duct 320. As a result, the steam supply portion 800 may be prevented from impeding the movement of air flowing through the circulation duct 320 or heating the air.

The steam supply portion 800 may be located apart from the inlet 362. For example, the steam supply portion 800 may be located rearward of the inlet unit 362.

The machine room 300 may include a fan installation portion 350 through which the circulation duct 320 and the inner case 200 are provided to communicate with each other. The fan installation portion 350 may include a blowing fan 353 that provides power to move the air inside the circulation duct 320 in one direction, and a fan housing 351 that accommodates the blowing fan 353 and is coupled to the circulation duct 320 or extends.

The fan installation portion 350 may include an exhaust duct 352 through which the circulation duct 320 and the exhaust hole 232 communicate with each other.

The exhaust duct 352 may be provided in the fan housing 351 with a cross-sectional area corresponding to the exhaust hole 232 and may extend toward the exhaust hole 232.

As a result, the air inside the inner case 200 may flow in through the base cover 360, may pass through the circulation duct 320, and may then be supplied back into the inner case 200 through the fan installation portion 350.

The base portion 310 may include a compressor installation portion 313 in which the compressor 342 that supplies refrigerant to the heat exchangers 341 and 343 is installed. The compressor installation portion 313 may be located outside the circulation duct 320.

A controller 700 that controls the clothing treatment apparatus may be installed on the base portion 310.

The base portion 310 may include a controller installation portion 312 that defines a space into which the controller is to be inserted below the circulation duct 320.

The controller may control all electronically controlled electrical components, such as the compressor 342, the steam supply portion 800, and the blowing fan 353.

The controller 700 is inserted and supported in the base portion 310, and thus vibration generated from the moving hanger 1000 or the laundry may be blocked from being transmitted to the controller 700 or may be attenuated and transmitted to the controller 700.

The controller 700 may be installed in the base portion 310 and thus may prevent noise or control errors from occurring due to a close separation distance from all electrical equipment installed in the machine room 300.

In the clothing treatment apparatus, the steam supply portion 800 is located on the upper side of the circulation duct 320, and the controller 700 is located at the lower side of the circulation duct 320. Therefore, the circulation duct 320 may be provided in a shape of a straight duct between the steam supply portion 800 and the controller 700. As a result, flow resistance of air passing through the circulation duct 320 may be minimized.

The circulation duct 320, the outside air duct 370, the steam supply portion 800, the controller 700, and the heat supply portion 340 may all be installed in the base portion 310, and thus the base portion 310 may be provided as one module. As a result, the base portion 310 may be retracted into and extended out of the machine room 300 forward or backward, allowing most electrical components to be easily installed, maintained, and repaired.

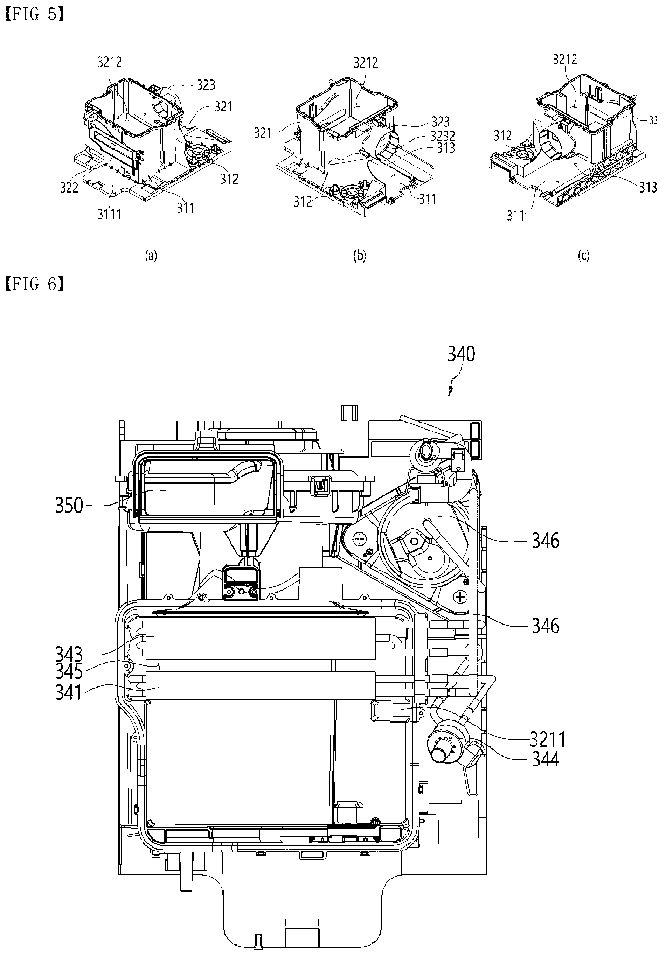

FIG. 5 shows a machine base structure of a clothing treatment apparatus.

FIG. 5(a) is a perspective view of the base portion 310 viewed from the front, and FIG. 5(b) and 5(c) are perspective views of the base portion 310 viewed from the rear.

The base portion 310 may be installed on a base plate that defines a lower surface of the clothing treatment apparatus. The base portion 310 may itself define the lower surface of the clothing treatment apparatus.

The base portion 310 may include a base bottom portion 311 that defines a support surface. The base bottom portion 311 may define the lower surface of the clothing treatment apparatus. The base bottom portion 311 may be installed on an upper surface of a bottom surface of the cabinet 100, which defines the lower surface of the clothing treatment apparatus.

The base portion 310 may be integrally provided with the circulation duct 320, which defines at least a portion of a flow path through which air moves. The circulation duct 320 may be formed to extend upward from the base bottom portion 311.

The circulation duct 320 may include a duct body 321 that extends from the base bottom portion 311 to define a flow path, a heat exchanger installation portion 3212 that provides a space in which an evaporator 341 or a condenser 343 is installed, and an air discharger 323 provided at a rear side of the duct body 321 through which air of the duct body 321 is discharged.

The air discharger 323 may be provided in a shape of a pipe extending rearward from the duct body 321. The diameter of the air discharger 323 may be less than the width of the duct body 321.

The air discharger 323 may be connected to the fan housing 350. The air discharged from the air discharger 323 may be guided into the inner case 200 through the fan housing 350.

The circulation duct 320 may include an outside air intake portion 322 formed to pass through a front surface of the duct body 321.

The outside air intake portion 322 may be provided to communicate with the outside air duct 370. The outside air duct 370 may be accommodate and supported on the front side of the outside air intake portion 322.

The circulation duct 320 may include a damper that opens and closes the outside air intake portion 322. It may be possible to allow or block outside air from flowing into the circulation duct 320 by opening and closing the damper.

The base portion 310 may include a compressor installation portion 312 that provides a space in which the compressor 342 is installed. The compressor installation portion 312 may be formed on one side of the base bottom portion 311 and may be formed integrally with the base bottom portion 311.

The compressor installation portion 312 may be formed with a protrusion for supporting the compressor 342. The compressor installation portion 312 may be located biased toward the rear side of the base portion 310. The compressor installation portion 312 may be located such that at least a portion of the compressor installation portion 312 overlaps the air discharger 323 in a width direction.

The compressor installation portion 312 may include a buffer member installed therein and reducing vibration transmitted from the compressor 342. The buffer member may be fixed to the protrusion.

The base portion 310 may include a controller installation portion 313 in which the controller 700 is installed. The controller installation portion 313 may be formed between the base bottom portion 311 and the circulation duct 320. The controller installation portion 313 may be formed between the base bottom portion 311 and a bottom surface of the circulation duct 320. The controller installation portion 313 may be provided in a shape of a duct that is open at either a front side or a rear side at a lower side of the circulation duct 320.

FIG. 6 shows a circulation duct structure of a clothing treatment apparatus.

The circulation duct 320 may extend from a base bottom portion upward to form a flow path through which air flows. The circulation duct 320 may include a heat exchanger installation portion 3212 that provides a space in which an evaporator 341 and a condenser 342 are installed. The heat exchanger installation portion 3212 may be provided inside the duct body 321.

The duct body 321 may be provided with an open upper surface. The condenser 343 and the evaporator 341 may be installed through the opening of the duct body 321.

The opening of the duct body 321 may be shielded by the base cover 360, and the base cover 360 and the duct body 321 may define a flow path of a circulation flow path 320.

A front surface of the duct body 321 may be spaced rearward from a front end of the base bottom portion 311.

As a result, the base bottom portion 311 may ensure a support surface 3111 on which one or more of the water supply tank 30, the drain tank 40, and the outside air duct 370 described above are installed and supported.

The heat supply portion 340 may include the evaporator 341 installed inside the circulation duct 320 and provided as a heat exchanger to cool and dehumidify air flowing into the circulation duct 320, the condenser 343 provided as a heat exchanger that heats air passing through the evaporator 341 to form hot air, the compressor 342 located outside the circulation duct 320 and supplying a refrigerant that exchanges heat with the air to the condenser 343, and an expansion valve 344 that expands and cools the refrigerant passing through the condenser 343.

As the duct body 321 is integrally molded with the base portion 310, the height of the heat exchanger installation portion 3212 may also be increased, and the height of the condenser 343 and the evaporator 341 may also be increased. As a result, the width of the condenser 343 and the evaporator 341 in front and rear directions may be reduced, and the number of refrigerant pipes passing through the condenser and the evaporator may be reduced. Accordingly, there is an effect of reducing a flow loss of air passing through the condenser and the evaporator.

Meanwhile, the sum of the lengths of the evaporator 341 and the condenser 343 may be less than the length of the heat exchanger installation portion 3212. Accordingly, the length of the heat exchanger installation portion 3212 in front and rear directions may be equal to or less than half the length of the duct body 321.

Therefore, the heat exchanger installation portion 3212 may be sufficiently spaced apart from the outside air intake portion 322, and thus a sufficient space through which outside air and air inside the inner case 200 may flow may be ensured inside the circulation duct 320.

An installation partition 3211 that separates the heat exchanger installation portion 3212 from the outside of the heat exchanger installation portion 3212 may be provided inside the duct body 321. The installation partition 3211 may protrude from a lateral surface of the duct body 321 to support the front side of the evaporator 341.

The duct body 321 may be expanded in width based on the installation partition 3211 and extend rearward.

As a result, the width of the heat exchanger installation portion 3212 may be greater than half the width of the base portion 310. The width of the circulation duct 320 may be greater than half the width of the base portion 310.

The width of the condenser 343 and the width of the evaporator 341 may also be greater than half the total width of the base portion 310. As described above, when the widths of the condenser 343 and the evaporator 341 are ensured, there is an effect of ensuring sufficient heat exchange capacity.

The fan housing 350 may be located to overlap the condenser 343 or the evaporator 341 in front and rear directions. Accordingly, air passing through the evaporator 341 and the condenser 343 may be introduced into the fan housing 350 without bending the flow path. In other words, the air flowing into the circulation duct 320 has an effect of minimizing flow loss because the flow path is not bent in a process of moving to the fan housing. The clothing treatment apparatus may further include a temperature sensor S1 that detects the temperature inside the inner case 200 or the temperature of air flowing into the circulation duct 320 from the inside of the inner case 200, and a refrigerant sensor S2 that detects the temperature of the refrigerant circulating the heat supply portion.

The temperature sensor S1 may be installed on an inner wall of the circulation duct 320, and the refrigerant sensor S2 may be provided at a discharge side of the compressor 342.

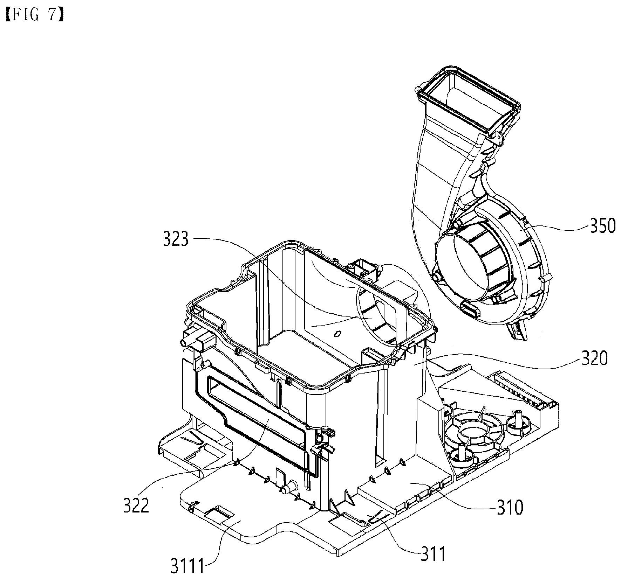

FIG. 7 shows the structure of the air discharger 323 of the clothing treatment apparatus.

The base portion 310 may include the air discharger 323 that discharges processed air toward the fan housing.

The air discharger 323 may allow the inside of the circulation duct 320 or the duct body 321 to communicate with the fan housing 350. The air discharger 323 may be provided in a shape of a bell mouth. The air discharger 323 may be provided in a shape of a bell mouth to reduce air flow loss and improve air circulation efficiency.

An air discharge pipe 3232 of the air discharger 323 may be provided in a shape of a pipe, and during a mold removal process based on a parting line 3233, a mold located before the parting line 3233 may be pulled forward, and a mold located behind the parting line 3233 may be pulled out rearward.

The fan installation portion 350 may be supported by being coupled to the air discharge pipe 3232. The fan housing 351 may have a coupling hole coupled to an outer peripheral surface of the air discharge pipe 3232, and the blowing fan 353 may be located in the coupling hole.

The fan housing 351 may include the exhaust duct 352 extending from the outer peripheral surface or the outside of the blowing fan 353 to the exhaust hole 232.

The fan housing 351 and the exhaust duct 352 may accommodate the blowing fan 353 therein and define a flow path through which air may move.

A motor that rotates the blowing fan 353 may be coupled to and supported on the outside of the fan housing 351.

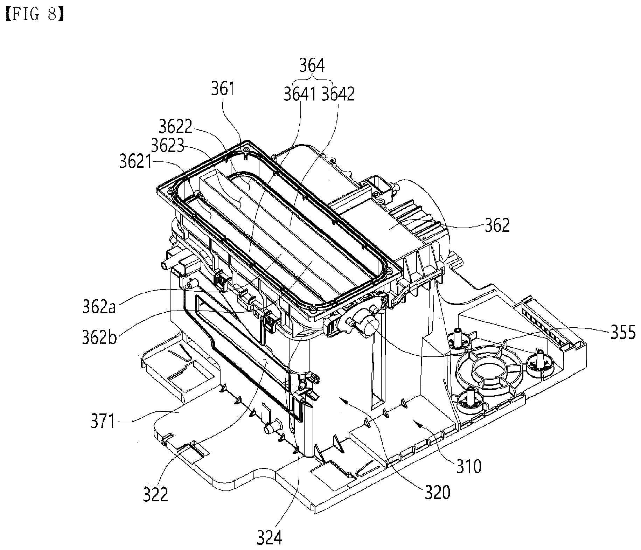

FIG. 8 shows a structure of a base cover of a clothing treatment apparatus.

The base cover 360 may be coupled to the upper surface of the circulation duct 320 to prevent the inside of the circulation duct 320 from being exposed.

The base cover 360 may include an inlet body 361 that is coupled to the upper surface of the circulation duct 320 and through which the inner case 200 communicates with the circulation duct 320, and a shielding body 363 that extends from the inlet body 361 to shield the circulation duct 320.

The inlet body 361 may be provided in a duct shape to allow the inlet hole 231 of the inner case to communicate with the inside of the circulation duct 320. The inlet body 361 may protrude further upward than the shielding body 363.

The inlet body 361 may be located ahead of the evaporator 341 so as not to face the evaporator 341 and the condenser 343 and may be placed ahead of the partition 3211.

The inlet body 361 may function as an inlet duct that moves air in the inner case 200 to the circulation duct 320.

The inlet body 361 may include the inlet 362 therein through which the air of the inner case 200 may pass.

In detail, the base cover 360 may include a first rib 362a extending in a width direction of the inlet body 361, and a second rib 362b spaced rearward from the first rib 362a and extending in the width direction.

The first rib 362a and the second rib 362b may be provided in parallel to each other. The first rib 362a and the second rib 362b may be provided in a plate shape extending in a vertical direction and may each have a height corresponding to the height of the inlet body 361.

A front edge of the inlet body 361 and the first rib 362a may define a first inlet 3621, the first rib 362a and the second rib 362b may define a second inlet 3622, and the second rib 362b and a rear edge of the inlet body 361 may define a third inlet 3623.

The first inlet 3621 and the third inlet 3622 may have the same area, and the second inlet 36222 may have an area less than that of the first inlet 3621 and the third inlet 3622.

The base cover 360 may include a damper portion 364 opening and closing the inlet 362, and a driver 365 that is coupled to the damper portion 364 and controls opening and closing of the damper portion 364.

The damper portion 364 may include a first damper portion 3641 opening and closing the first inlet 3621, and a second damper portion 3642 opening and closing the third inlet 3623.

The first damper portion 3641 may be provided in a plate shape with an area corresponding to the first inlet 3621, and may be rotatably coupled to both lateral surfaces of the inlet body 361 inside the first inlet 3621.

The second damper portion 3642 may be provided in a plate shape with an area corresponding to the third inlet 3622, and may be rotatably coupled to both lateral surfaces of the inlet body 361 inside the third inlet 3622.

The second inlet 3622 may include a blocking filter 366 that allows air to pass through but filters foreign substances such as fine dust and lint.

The blocking filter 366 may be inserted into the second inlet 3622 to partition the first inlet 3621 and the third inlet 3623. The blocking filter 366 may be located to extend from the second inlet 3622 to be in contact with the bottom surface of the circulation duct 320.

The blocking filter 366 may be provided as a filter for filtering even moisture. For example, the blocking filter 366 may be provided as a HEPA filter or the like.

When the blocking filter 366 is inserted into the second inlet 3622, a shielding member that shields the second inlet 3622 may be further coupled.

The driver 365 may include a motor that provides power to selectively rotate the first damper portion 364 and the second damper portion 365, and a plurality of gear members that selectively rotate the first damper 364 and the second damper 365 while being engaged with the motor and rotating.

Due to the driver 365, the first inlet 3611 and the third inlet 3623 may be selectively opened.

Due to the driver 365, the air contained inside the inner case 200 may flow into the circulation duct 320 along the first inlet 3621 or may flow into the circulation duct 320 along the third inlet 3623.

The driver 365 may control the first damper portion 3641 and the second damper portion 3642 to open both the first inlet 3611 and the third inlet 3623 and control the first damper portion 3641 and the second damper portion 3642 to close both the first inlet 3611 and the third inlet 3623.

The driver 365 may be provided in any structure as long as the driver 365 may rotate the first damper portion 3641 and the second damper portion 3642. For example, the driver may be provided as a combination of a motor, a main gear rotating by the motor, and a driven gear coupled to the first damper portion and the second damper and rotating by rotation of the main gear.

The base cover 360 may include the shielding body 363 that extends from the inlet body 361 and shields the evaporator 341 and the condenser 343. The shielding body 363 may be provided in a plate shape.

The base cover 360 may be detachably coupled to an upper surface of the circulation duct 320 through an inlet hook 3612 extending from the lower surface of the inlet body 361.

The circulation duct 320 may include a coupler that is detachably coupled to the inlet hook 3612.

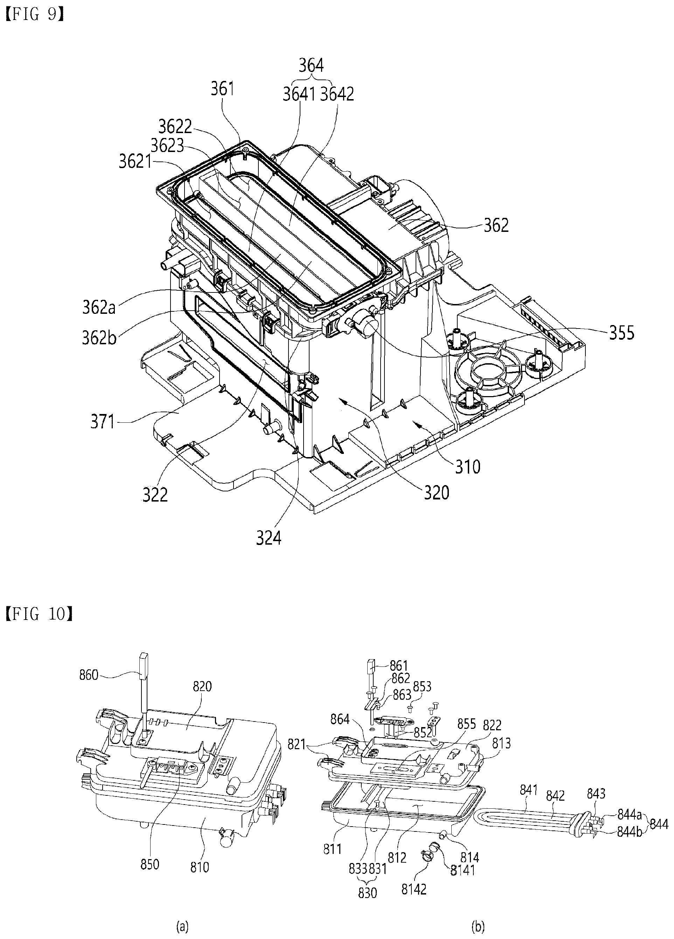

FIG. 9 shows an installation structure of a steam supply portion.

The steam supply portion 800 may be accommodated and supported on the base cover 360.

The steam supply portion 800 may include a steam generator 810 that is accommodated on the base cover 360 and stores water for generating the steam.

The steam supply portion 800 may further include an installation bracket 870 for fixing the steam generator 810 to the base cover 360.

The installation bracket 870 may be coupled to the base cover 360 to fix the steam generator 810.

The installation bracket 870 may include a lower panel 871 supporting a lower surface of the steam generator 810 and a lateral panel 872 supporting both lower surfaces of the steam generator 810 on the lower panel 871.

The installation bracket 870 may further include one or more fixing clips 873 that extend from the lateral panel 872 to prevent the steam generator 810 from being separated.

The fixing clip 873 may be detachably provided on the upper side or lateral surface of the steam generator 810.

The compressor 342 may be located below the steam supply portion 800.

The installation bracket 870 may be configured to block heat generated from the compressor or heat generated from the refrigerant compressed in the compressor from being transmitted to the steam supply portion 800.

The installation bracket 870 may also block fire from spreading to the steam supply portion 800 when a fire occurs in the compressor 342.

The base cover 360 may include a fastener 3631 provided on the shielding body 363 and detachably coupled to the steam supply portion 800. The fastener 3631 may be provided in a structure that is detachably coupled to a protrusion protruding from a lower side of the steam generator 810.

Accordingly, even if a large amount of water is contained within the steam generator 810, the steam generator 810 may be stably accommodated on the base cover 360.

The steam generator 810 is located above the circulation duct 320 and a distance to the inner case 200 is further reduced, and thus condensation of steam generated by the steam generator 810 before reaching the inner case 200 may be minimized.

FIG. 10 shows a detailed structure of a steam supply portion.

Referring to FIG. 10(a), the steam supply portion 800 may include the steam generator 810 for receiving and storing water to generate steam, and a steam heater 840 accommodated in the steam generator 810 to heat the water to generate steam.

The steam generator 810 may be provided in a case shape that defines a space for accommodating the steam heater 840.

For example, the steam generator 810 may be provided in the form of a case with an open top and may be provided to accommodate the steam heater 840.

The steam supply portion 800 may further include a case cover 820 that is coupled to the steam generator 810 to prevent the steam heater 840 from being exposed to the outside and to prevent water from leaking.

The case cover 820 may include a water level sensor 850 that detects a water level of the steam generator 810, and a steam sensor 860 that detects the temperature inside the steam generator 810 or whether steam is generated inside the steam generator 810.

Referring to FIG. 10(b), the steam generator 810 may include a case body 811 that stores the water and provides a space to accommodate the steam heater 840.

The case body 811 has an open top such that various components are easily installed inside the case body 811.

The case body 811 may include a heater insertion hole 8111 with penetrated one side through which the heater 840 is to be inserted or extracted.

The case body 811 may include a recovery pipe 814 that receives water to generate steam.

The recovery pipe 814 may discharge water contained within the case body 811 to the outside.

The recovery pipe 814 may include a shielding clip 8142 that maintains the recovery pipe 814 in a closed state to be opened only when residual water inside the steam generator 810 is removed and maintains the shielding stopper 8141 to be coupled to the recovery pipe 814 to prevent the shielding stopper 8141 from being separated arbitrarily.

Accordingly, when repairing the steam generator 800 or preventing freezing or bursting of the steam generator 800, water inside the steam generator 800 may be discharged through the recovery pipe 814.

The recovery pipe 814 may be provided to receive water from the water supply tank 30. Details are described later.

A heater fixer 830 for supporting or fixing the steam heater 840 may be installed inside the case body 811. The heater fixer 830 may include a fixing clip 831 for fixing the steam heater 840, and a clip fastener 833 for fixing the support clip 831 to the case body 811.

The fixing clip 831 may be provided to accommodate or surround at least a portion of the steam heater 840.

The steam supply portion 800 may include a water supply pipe 815 through which water is supplied. The water supply pipe 815 may communicate with the water supply tank 30 to receive water.

The water supply pipe 815 may be provided on the case cover 820 or may be located above the steam generator 810. As a result, water may be prevented from flowing back through the water supply pipe 815.

The steam supply portion 800 may include a steam pipe 813 that discharges steam generated by driving the steam heater 840 to the outside. The steam pipe 813 may also be provided above the case cover 820 to prevent water from arbitrarily being discharged into the steam pipe 813.

The steam pipe 813 may communicate with the steam hole 233 of the inner case 200.

The case cover 820 may include a water level sensor hole 854 in which the water level sensor is to be installed.

The water level sensor 850 may include one or more contact protrusions 852 that are inserted into the water level sensor hole 854 and are immersed in water to detect a water level, and a sensor body 851 that is coupled to the water level sensor hole 854 or supported on the case cover 820 to maintain the contact protrusion 852 in a floating state inside the steam generator 810.

The sensor body 851 may be coupled to the case cover 820 through a sensor fastening member 853.

The case cover 820 may include an insertion hole 864 into which the steam sensor 860 is to be installed. The steam sensor 860 may include a detection device 861 that is inserted into the insertion hole 864 and detects whether steam is generated inside the steam generator 810, a support 863 that fixes the detection device 861 to the case cover 820, and a coupling member 862 that couples the support 863 to the case cover 820.

The detection device 861 may be provided as a humidity sensor or a temperature sensor and may detect whether steam is generated inside the steam generator 810.

The case cover 820 may include a cover hook 821 that extends forward and is to be coupled to the base cover 860.

A fixing protrusion 822 for fixing the lower side of the inner case 200 or a separate steam discharger 900 may be provided at a rear side of the case cover 820.

The steam heater 840 may be inserted into the heater insertion hole 8111, may be accommodated in the steam generator 810, and may heat water by receiving power.

The steam heater 840 may be provided as a sheath heater, or the like and may also be controlled by the controller 700 to be repeatedly driven and stopped.

The steam heater 840 may include a first heater 841 that receives first power to heat water and a second heater 842 that receives power less than the first power to heat water.

As a result, the second heater 842 may be provided to heat a smaller amount of water and generate more steam than the first heater 841.

The first heater 841 and the second heater 842 may be configured to divide and consume the maximum heater electrical energy allowed for the steam heater 840 in the clothing treatment apparatus. That is, when the first heater 841 is configured to consume a portion of the maximum heater electrical energy, the second heater 842 may be provided to consume the remaining amount of the maximum heater electrical energy.

For example, when the general maximum heater electrical energy allowable for the steam heater 840 is 1500 w, the first heater 841 may be provided to consume 880 w, and the second heater 842 may be provided to consume 600 w. As such, 20 w may be distributed less in consideration of errors.

The steam heater 840 may include three or more heaters. For example, the steam heater 840 may include the first heater 841, the second heater 842, and the third heater 843, and the first heater 841, the second heater 842, and the third heater 843 may be provided to divide and consume the maximum heater electrical energy.

Hereinafter, the steam heater 340 will be described based on that the steam heater 340 includes the first heater 841 and the second heater 842.

The first heater 841 and the second heater 842 may be formed as a U-shaped metal tube.

The steam heater 840 may include a heater sealer 843 fixing the first heater 841 and the second heater 842 and sealing the heater through hole 8111 and a terminal portion 844 that supplies current to the first heater 841 and the second heater 842.

The terminal portion 844 may include a first terminal 844a that supplies current to the first heater 841 and a second terminal 844b that supplies current to the second heater 842.

The first heater 841 and the second heater 842 may be located at the same height. Accordingly, the first heater 841 and the second heater 842 may be provided to generate steam by heating water at the same water level.

Accordingly, the controller 700 may adjust the amount of steam generated by using both or selectively the first heater 841 and the second heater 842 and the amount of power consumed.

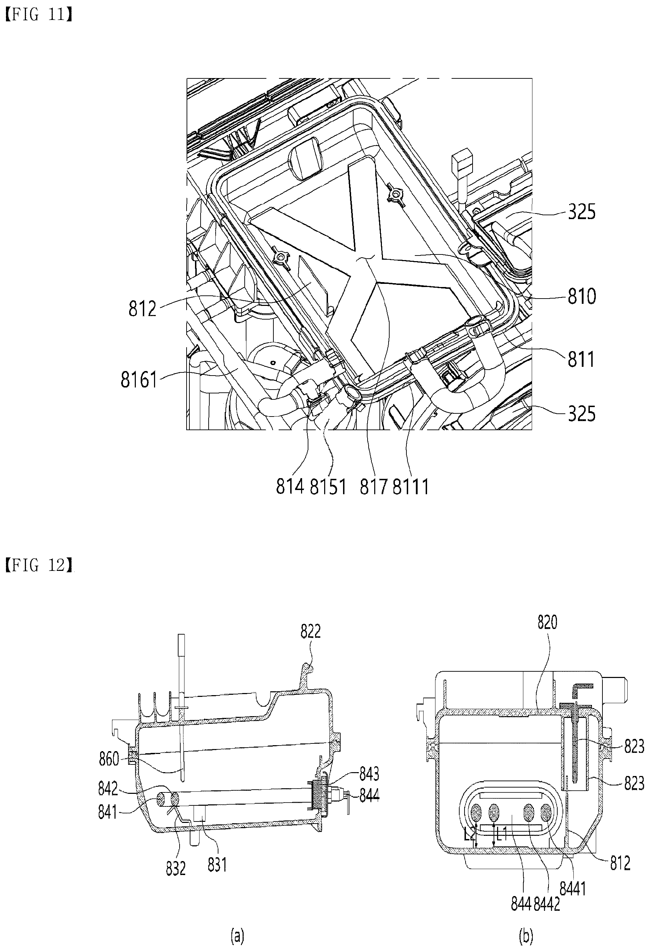

FIG. 11 shows the inside of the steam generator.

The steam generator 810 may include a heating space 817 that stores water therein and accommodates the steam heater 840.

The steam generator 810 may receive water to generate steam through a water supply hose 8151 connected to the water supply pipe 815.

The steam generated by driving the steam heater 840 in the steam generator 810 may be discharged to the outside of the steam supply portion 800 along the steam hose 8131 through the steam discharge pipe 813.

The steam hose 8131 may communicate with the steam hole 233 of the inner case 200.

The steam generator 810 may include a separation partition 812 separating the heating space 817 and the water level sensor 850 from each other. That is, the separation partition 812 may be provided to separate the steam heater 840 and the water level sensor 850 from each other and may be located to be biased to one side of the case body 811.

The water level sensor 850 may be located between the separation partition 812 and an inner surface of the case body 811.

As a result, it may be possible to prevent water generated when the water boils from being transmitted to the water level sensor 850 and prevent heat generated from the steam heater 840 from being directly transmitted to the water level sensor 850.

FIG. 12 shows a steam supply portion in which the steam heater is installed.

Referring to FIG. 12(a), the steam sensor 860 may be located above the steam heater 840 and may be provided to detect the temperature inside the steam generator 810. Accordingly, the steam sensor 860 may detect that steam is generated when the water reaches 100 degrees or a high temperature.

The steam heater 840 may be supported by the support clip 832 to prevent the steam heater 840 from being in contact with the bottom surface of the steam generator 810 or the like.

In addition, an upper side of the steam heater 840 may be surrounded by the fixing clip 831 to prevent a water level at which the steam heater 840 is located from changing.

Referring to FIG. 12(b), a heater sealer 844 of the steam heater 840 may include a first support hole 8441 through which the first heater 841 passes to be supported and a second support hole 8442 through which the second heater 842 passes to be supported.

A first length L1 by which the first support hole 8441 is spaced apart from the bottom surface of the steam generator 810 and a second length L2 by which the second support hole 8442 is spaced apart from the bottom surface of the steam generator 810 may be equal to each other.

Even though the diameters of the first support hole 8441 and the second support hole 8442 are different, an installation height may be the same.

Accordingly, the first heater 841 and the second heater 842 may generate steam by heating water at the same water level.

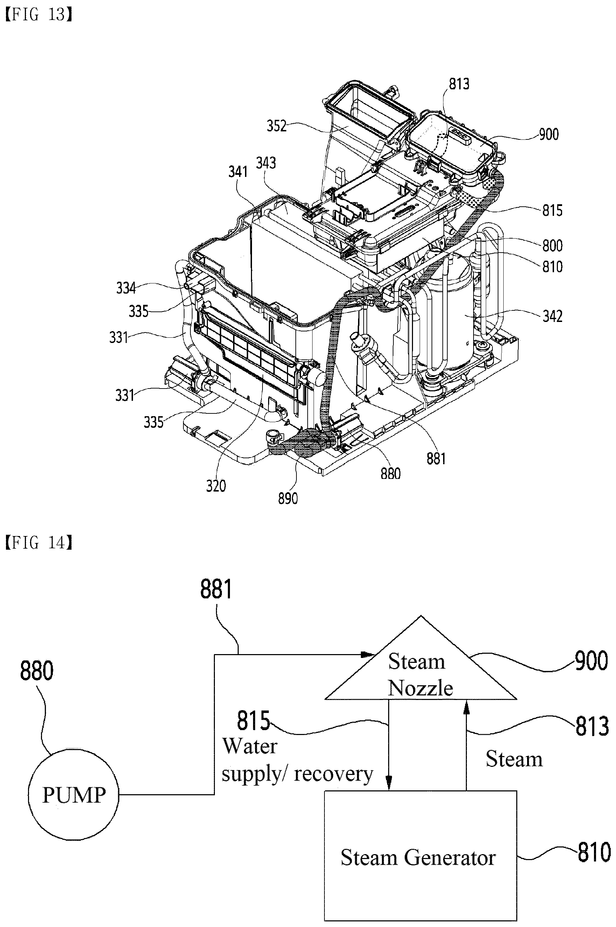

FIG. 13 shows in detail a steam supply portion structure of a clothing treatment apparatus.

Steam generated inside the steam generator 810 may be directly supplied into the inner case 200 through the steam pipe 813.

However, when steam is directly supplied into the inner case 200 through the steam pipe 813, there is a risk that water contained in the steam generator 810 is also supplied into the inner case 200.

There is a risk that water heated in the steam generator 810 heats the bottom surface of the inner case 200, causing thermal damage to the inner case 200.

To prevent this, the steam supply portion 800 may further include a steam nozzle 900 that receives steam generated from the steam generator 810 and supplies the steam to the inner case 200.

The steam nozzle 900 may be spaced apart from the steam generator 810, and may be provided to receive only the steam formed in the steam generator 810 and not to receive water contained in the steam generator 810.

For example, the steam nozzle 900 may be located above the steam generator 810 and connected to the steam pipe 813. Accordingly, the steam generated in the steam generator 810 may rise due to a density difference and may be supplied to the steam nozzle 900 along the steam pipe 813, and the water contained in the steam generator 810 may not flow in to the steam pipe 813 or the steam nozzle 900 by gravity.

The steam nozzle 900 may be located between the bottom surface of the inner case 200 and the steam generator 810 and may be provided to communicate with the steam hole 233.

The steam supply portion 800 is provided to generate steam by receiving water from the water supply tank 30.

To this end, the clothing treatment apparatus may further include a water supply pump 880 that supplies water contained in the water supply tank 30 to the steam supply portion 800.

The steam supply portion 800 may further include a supply pipe 890 for guiding the water contained in the water supply tank 30 to the water supply pump 880.

The clothing treatment apparatus may further include the water supply pipe 815 that guides the water discharged from the water supply pump 880 to the steam supply portion 800. The water supply pipe 815 may be provided in the form of the water supply hose 8151 such as rubber.

As a result, the water supply pump 880 may be provided to receive water from the supply pipe 890 connected to the water supply tank 30. The water supply pump 880 may be provided to discharge water to supply water to the steam supply portion 800 through a water supply pipe 881.

It may be seen that the steam supply portion 800 further includes the water supply pump 880 that provides power to supply water contained in the water supply tank 30 to the steam generator 810.

The supply pipe 890 may be provided as a hose connecting the water supply tank 30 and the water supply pump 880 to each other, and the water supply pipe 881 may be provided as a hose coupled to the water supply pump 880.

The water supply pipe 815 may be provided to directly connect the water supply pump 880 and the steam generator 810 to each other. As a result, the water supplied from the water supply pump 880 may be directly supplied to the steam generator 810 and may be heated by the steam heater 840 to generate steam. The steam may be supplied to the steam nozzle 900 through the steam pipe 813 and delivered to the inner case 200.

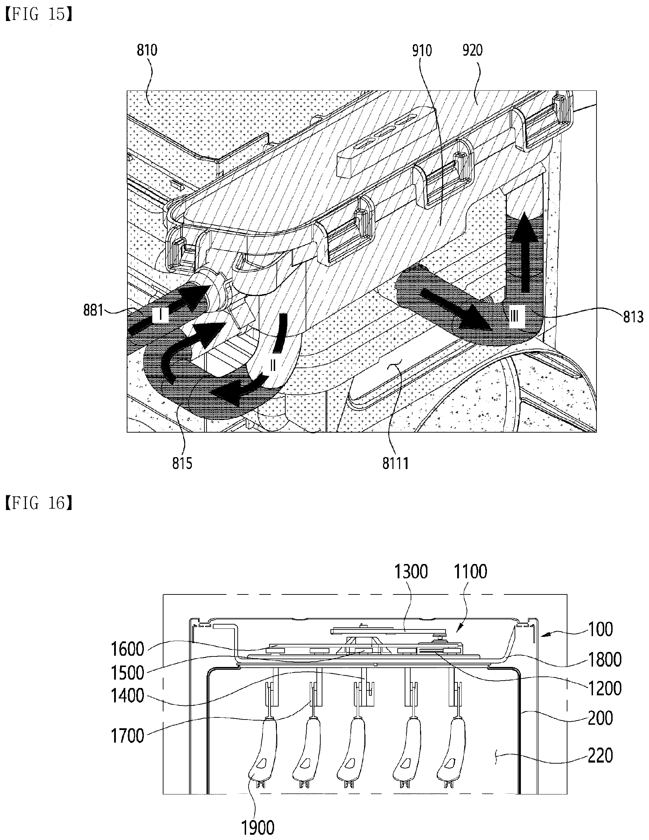

However, as shown in FIG. 15, the water supply pipe 815 may be provided to directly connect the water supply pump 880 and the steam nozzle 900 to each other.

In other words, the clothing treatment apparatus may be provided to supply water contained in the water supply tank 30 to the steam nozzle 900 rather than the steam generator 810. Water supplied from the water supply pump 880 may be directly supplied to the steam nozzle 900.

The water supply pump 880 may not be directly connected to the steam generator 810. It may be seen that the water supply pump 880 communicates with the steam generator 810 and the steam nozzle 900.

The steam nozzle 900 may receive water contained in the water supply tank 30 through the water supply pump 880. The water supplied to the steam nozzle 900 may be delivered to the steam generator 810.

The steam nozzle 900 may be located above the steam generator 810, and thus water supplied to the steam nozzle 900 may be automatically supplied to the steam generator 810.

As a result, the steam nozzle 900 may be provided to receive water contained in the water supply tank 30 through the water supply pump 880 and deliver the water to the steam generator 810.

The steam supply portion 900 may further include a recovery pipe 815 connecting the steam nozzle 900 and the steam generator 810 to each other. The recovery pipe 815 may supply water that is supplied to and temporarily contained in the steam nozzle 900, to the steam generator 810.

Accordingly, the steam pipe 813 through which steam flows into the steam nozzle 900 and the recovery pipe 815 through which water is discharged from the steam nozzle 900 may be provided as separate flow paths.

Accordingly, water supplied from the steam nozzle 900 may be prevented from interfering with movement of steam supplied from the steam generator 810.

The recovery pipe 815 may be provided in a U-shape. That is, a portion of an area between two ends of the recovery pipe 815 may be located lower in height than the two ends. As a result, a certain amount of water may be accumulated in the recovery pipe 815, like a water trap, thereby preventing re-inflow of steam or water supplied from the steam generator 810 through the recovery pipe 815.

The steam pipe 813 may include the steam hose 8131 connecting the steam nozzle 900 and the steam generator 810 to each other. The steam hose 8131 may include rubber.

The recovery pipe 815 may be provided separately from the steam pipe 813 and may be spaced apart from the steam pipe 813. The recovery pipe 815 may include a recovery hose 8151 connecting the steam nozzle 900 and the steam generator 810 to each other. The recovery hose 8151 may include rubber.

The steam generator 810 may receive and accommodate water supplied to the steam nozzle 900. The steam generator 810 is not directly connected to the water supply pump 880, and thus there is no possibility that water flowing into the steam generator 810 may flow back to the water supply pump 880.

The steam nozzle 900 is located above the steam generator 810, and the steam nozzle 900 is connected to the steam generator 810 through the recovery pipe 815, and thus water supplied to the steam nozzle 900 may be automatically supplied to the steam generator 810 by gravity. As a result, the water supplied from the water supply pump 880 to the steam nozzle 900 is unlikely to flow back to the water supply pump 880.

As a result, a check valve between the water supply pump 800 and the steam nozzle 900 or the steam generator 810 may be omitted. Therefore, the water supply pipe 881 may be provided as a single water supply hose. A flow path for supplying water to the steam supply portion 900 may be simplified, and a possibility of water leaking from the flow path may be correspondingly reduced.

A large amount of water may be supplied to the steam nozzle 900 through the water supply pump 800 at considerable pressure. As a result, foreign substances or growing bacteria accumulated in the steam nozzle 900 may be washed away with the water supplied to the steam nozzle 900 and washed down into the steam generator 810. Accordingly, the steam nozzle 900 may always be maintained in a clean state, and a flow of supplied steam may be prevented from being disturbed.

When the steam supplied from the steam nozzle 900 is condensed, the condensed water may reflow into the steam generator 810 through the recovery pipe 815. That is, the condensed water generated from the steam nozzle 900 may be recovered to the steam generator 810 in the same direction of movement of the water supplied from the water supply pump 800, and may be recycled as water for generating the steam. Therefore, clothing treatment apparatus may prevent a water level in the water supply tank 30 from being rapidly lowering and also prevent waste of water.

FIG. 14 is a schematic diagram of a flow path structure of a steam supply portion.

Water supplied from the water supply pump 880 may be supplied to the steam nozzle 900 along the water supply pipe 881.

The steam nozzle 900 may supply supplied water to the steam generator 810.

The steam generator 810 may receive water through the recovery pipe 815.

When water is heated by the steam heater 840 in the steam generator 810 to generate steam, the steam may be supplied to the steam nozzle 900 along the steam pipe 813.

In this case, the recovery pipe 815 and the steam pipe 813 may be separated from each other and located in the steam generator 810.

The steam condensed in the steam nozzle 900 may be re-introduced into the steam generator 810 through the recovery pipe 815.

The steam generator 810 may omit hoses connected to the outside other than the recovery pipe 815 and the steam pipe 813 connected to the steam nozzle 900. That is, the water supply pump 880 may not be directly connected to the steam generator 810, and thus the hose connecting the water supply pump 800 and the steam generator 810 to each other may be omitted.

Accordingly, the clothing treatment apparatus only needs to have the water supply pipe 881, the recovery pipe 815, and the steam pipe 813 as a flow path, and thus a flow path structure may be simplified.

The steam nozzle 900 may be located above the steam generator 810. All of the water supplied to the steam nozzle 900 may be guided to the steam generator 810 without a separate check valve.

Therefore, when the water supplied from the water supply pump 880 is supplied to the steam nozzle 900, all of the water supplied to the steam nozzle 900 is guided to the steam generator 810, and thus the possibility of water flowing back from the water supply pump 880 from the steam nozzle 900 may be blocked.

Therefore, a check valve between the water supply pump 880 and the steam nozzle 900 may be omitted.

The check valve may not need to be installed in the water supply pipe 880, and thus the water supply pipe 880 may not need to be provided in plural pieces and may be provided as a single hose. As a result, not only is the flow path structure more simplified, but installation and repair of the flow path are also simplified, and the possibility of water leaks may be further reduced.

FIG. 15 shows a flow path structure of a steam nozzle.

The steam nozzle 900 may include a supply container 910 that receives steam from the steam case 810 or receives and accommodates water from the water pump 880.

The supply container 910 may be provided in a case shape with an internal space for receiving and accommodating water or steam.

The water supply pipe 881 may be connected to the supply container 910 at a higher level than the bottom surface of the supply container 910. For example, the water supply pipe 881 may be coupled to a lateral surface of the supply container 910. As a result, the water supplied to the supply container 910 may be prevented from flowing back into the water supply pipe 881.

The water supply pipe 881 may be connected to the supply container 910 at a higher level than recovery pipe 815. For example, the recovery pipe 815 may have one end coupled to the bottom surface of the supply container 910 and the other end coupled to the steam generator 810. Accordingly, the water supplied to the water supply pipe 881 may automatically flow into the recovery pipe 815.

The recovery pipe 815 may be provided in a U-shape to accommodate a certain amount of water therein.

The recovery pipe 815 may be located adjacent to the water supply pipe 881. As a result, the water supplied to the water supply pipe 811 may quickly flow into the steam case 810 without remaining in the supply container 810.

The steam pipe 813 may have one end coupled to a lower side of the supply container 910 and the other end coupled to an upper end of the steam generator 810 or the steam cover 820. Accordingly, the steam generated from the steam generator 810 may be automatically supplied to the supply container 910 due to a density difference.

The steam pipe 813 may be connected to a higher position than the recovery pipe 815 in the supply container 910. The steam pipe 813 may be located farther from the water supply pipe 881 than the return pipe 815.

As a result, more water supplied to the supply container 910 may be supplied to the recovery pipe 815 without flowing back into the steam pipe 813.

The steam pipe 813 may be coupled to the bottom surface of the supply container 910.

The bottom surface of the supply container 910 may have a lower portion at which the recovery pipe 815 is connected and a higher portion at which the steam pipe 813 is connected. To this end, a step may be formed on the bottom surface of the supply container 910 or may be provided at an angle.

The steam nozzle 900 may further include a container cover 920 coupled to an upper side of the supply container 910. The container cover 920 may include a steam injection hole 923 provided to pass through an upper side. The steam injection hole 923 may be provided to communicate with the inside of the inner case 200.

The supply container 910 may be provided in the form of a box with an open top, and the container cover 920 may be provided to shield the upper side of the supply container 910.

Steam supplied through the steam pipe 813 may be discharged through the steam injection hole 923 and supplied into the inner case 200.

The steam pipe 813 may be located between the steam injection hole 932 and an inner surface of the supply container 910. The steam injection hole 932 may be located between the water supply pipe 881 and the steam pipe 813 and may be located between the recovery pipe 815 and the steam pipe 813. As a result, the condensed steam that may not be discharged through the steam injection hole 932 may be discharged into the recovery pipe 815 without remaining inside the supply container 910.

The steam injection hole 932 may be located at the center of the container cover 920 based on a width direction.

The container cover 920 may be coupled to the supply container 910 by hook coupling or the like. Accordingly, a separate fastening member that couples the container cover 920 and the supply container 910 may be omitted.

The supply container 910 is provided with an open top, and thus may be advantageous to install various shapes or structures therein, and the container cover 920 may be advantageous for installing various structures at a lower side.

Based on the steam injection hole 932, the water supply pipe 881 and the recovery pipe 815 are located at one side of the supply container 910, and the steam pipe 813 is located at the other side of the supply container 910.

That is, the water supply pipe 881 and the recovery pipe 815 are located adjacent to each other, and the steam pipe 813 may be located farther from the water supply pipe 881 than the recovery pipe 815.

Accordingly, water moving to the water supply pipe 881 may be supplied to the supply container 910 in direction I. The water supplied to the supply container 910 may be immediately discharged in direction II to the recovery pipe 815 and may be supplied to the steam generator 810. The steam supplied from the steam pipe 813 may be supplied to the supply container 910 in direction III and discharged through the steam injection hole 932, and the condensed water may be discharged into the recovery pipe 815 in direction II and re-supplied to the steam generator 810.

Hereinafter, an embodiment of the moving hanger 1000 will be described in detail.

The moving hanger 1000 may be provided to move linearly back and forth inside the inner case 200. As a result, the laundry may move back and forth in a straight line inside the inner case 200 and shake.

The moving hanger 1000 may be provided to periodically shake laundry at various frequencies.

For example, the moving hanger 1000 may be provided in a structure similar to Korean Patent Publication No. 10-1285890.

The moving hanger 1000 may be provided to shake the laundry while reciprocating and rotating the laundry inside the inner case.

That is, in the clothing treatment apparatus, the moving hanger 1000 may be provided in any structure as long as the laundry may be shaken in the inner case.

Hereinafter, a structure in which the moving hanger 1000 reciprocates and rotates the laundry will be described.

FIG. 16 shows an upper structure of an inner case of a clothing treatment apparatus.

The moving hanger 1000 of the clothing treatment apparatus may include a power transmission unit 1400 disposed on the upper side of the inner case 200 and provided to shake the clothes hanger 1900.

A hanger 1700 on which the clothes hanger 1900 is to be accommodated or mounted may be provided below the power transmission unit 1400.

As a result, when the power transmission unit 1400 moves, the hanger 1700 moves, and the clothes hanger 1900 mounted on the hanger 1700 shakes, resulting in an effect of shaking off the laundry.

The power transmission unit 1400 may be provided in plural numbers, and the hanger 1700 coupled to the power transmission unit 1400 may also be provided in plural numbers. As a result, a large amount of laundry corresponding to the number of power transmission units 1400 may be refreshed by being located inside the inner case 200.

The moving hanger 1000 may further include a driver 1200 that provides power to move the power transmission unit 1400.

The driver 1200 may be provided to be exposed inside the inner case 200 as long as the driver 1200 may transmit power to the power transmission unit 1400. However, the driver 1200 is provided to operate by receiving electric energy, and thus may be prevented from being exposed to steam or hot air.

Accordingly, the driver 1200 may be located between the upper surface of the inner case 200 and the cabinet 100 and may be prevented from being exposed to the receiving space 220.

The power transmission unit 1400 may receive power from the driver 1200 through the inner case 200 and transmit the power to the hanger 1700.

The power transmission unit 1400 may be formed to pass through the upper surface of the inner case 200 and extend into the receiving space 220. A lower end of the power transmission unit 1400 may be exposed to the receiving space 220, and an upper end of the power transmission unit 1400 may be exposed above the inner case 200.

The power transmission unit 1400 may be provided in a rod shape, tube shape, or plate shape, in which a length is greater than a thickness.

The upper surface of the inner case 200 may support loads of the power transmission unit 1400 and the driver 1200. laundry is mounted and moved on the power transmission unit 1400, and the load of the driver 1200 is relatively heavy. Therefore, to stably install the moving hanger 1000 on the upper surface of the inner case 200, the clothing treatment apparatus 1 may further include a support portion 1800.

The support portion 1800 may be disposed on the upper side of the inner case 200 and may be coupled to and supported by the cabinet 100. The support portion 1800 may include a metal material that is durable and difficult to deform.

The power transmission unit 1400 and the driver 1200 may be accommodated on the support portion 1800 and located on the upper side of the inner case 200. The power transmission unit 1400 may be formed to pass through the support portion 1800 and extend into the receiving space 220.

The driver 1200 includes a motor that rotates a rotation shaft. The driver 1200 may be provided to move the power transmission unit 1400 with power to rotate the rotation shaft.

However, it may be difficult to shake the power transmission unit 1400 with sufficient displacement just by rotating the rotation shaft in place.

Therefore, the moving hanger 1000 may further include a displacement generator 1300 that is coupled to the rotation shaft rotated by the motor and generates sufficient displacement (amount of change in position) to enable the power transmission unit 1400 to move.

The displacement generator 1300 may be connected or coupled to the driver 1200.

For example, the displacement generator 1300 may be provided to transmit power of the driver 1200 to the power transmission unit 1400.

The displacement generator 1300 may include an eccentric shaft that rotates along a trajectory larger than a diameter of the rotation shaft. The eccentric shaft may be directly coupled to the rotation shaft of the driver 1200 or may be eccentrically coupled or extended to a power shaft 1240 rotated by the rotation shaft.

The displacement generator 1300 may be provided in any structure as long as the displacement generator 1300 may generate a displacement that causes the power transmission unit 1400 to move back and forth over a certain range. The detailed structure is described later.

When the driver 1200 operates, the power generated from the rotation shaft may generate the displacement of the displacement generator 1300, and the power transmission unit 1400 may move according to the displacement of the displacement generator 1300.

The displacement generator 1300 may directly move the power transmission unit 1400, but may also move the power transmission unit 1400 through an additional configuration.

The moving hanger 1000 may be provided to reciprocate the power transmission unit 1400.

The moving hanger 1000 may be provided to rotate the power transmission unit 1400. In detail, the moving hanger 1000 may be provided to reciprocate and rotate the power transmission unit 1400 in a certain angular range rather than reciprocating in a straight line.

The power transmission unit 1400 may be provided to rotate clockwise or counterclockwise in a fixed position, and laundry mounted on the power transmission unit 1400 may also rotate clockwise or counterclockwise. The power transmission unit 1400 is provided to rotate by the moving hanger 1000, but a position of the power transmission unit 1400 may vary to the left or right and may not move.

Even if laundry rotates inside the inner case 200 due to the power transmission unit 1400, movement of the center of gravity within the inner case 200 may be restricted. Therefore, even when the moving hanger 1000 operates, vibration occurring inside the inner case 200 may be drastically reduced, and noise generation may be minimized.

To this end, the moving hanger 1000 may further include a reciprocating rotation unit 1500 that converts continuous rotational energy generated by the driver 1200 or the displacement generator 1300 into a reciprocating rotation movement of the power transmission unit 1400.

The reciprocating rotation unit 1500 may be provided to connect the displacement generator 1300 and the power transmission unit 1400 to each other. The reciprocating rotation unit 1500 may be provided to connect the displacement generator 1300 and the power transmission unit 1400 to each other at an upper level than the inner case 200. The reciprocating rotation unit 1500 may be prevented from being exposed to the receiving space 220, thereby preventing the laundry from being damaged by the reciprocating rotation unit 1500.

The moving hanger 1000 may be provided to reciprocate and rotate only one of the plurality of power transmission units 1400.

However, if only one power transmission unit 1400 rotates, laundry mounted on the rotated power transmission unit may collide with laundry mounted on the other power transmission unit 1400 and be damaged. There is a risk that impact may be transmitted to the moving hanger 1000 and the moving hanger 1000 may be damaged.

Therefore, the moving hanger 1000 may be provided to rotate all of the plurality of power transmission units 1400.

The moving hanger 1000 may rotate the plurality of power transmission units 1400 as one unit. The moving hanger 1400 may be provided to rotate a plurality of power transmission units 1400 at the same time at the same angle. As a result, the clothing treatment apparatus may prevent the power transmission units 1400 from colliding with each other.

It may be advantageous for the power generated by the driver 1200 to be directly transmitted to the plurality of power transmission units 1400 to rotate all power transmission units 1400.

However, when the driver 1200 is provided to directly transmit power to each power transmission unit 1400, a structure connecting the driver 1200 and all power transmission units 1400 to each other may become complicated.

When the driver 1200 is provided in plurality, or when a plurality of components connecting the driver 1200 to all power transmission units 1400 is provided, excessive loads may be applied to the inner case 200 or the support portion 1800. An inconvenience of having to control a plurality of drivers 1200 may also result.

When the displacement generator 1300 and the reciprocating rotation unit 1500 are connected to transmit power transmitted from one driver 1200 to all power transmitting units 1400, the arrangement and structure of the displacement generator 1300 and the reciprocating rotation unit 1500 may become complicated, resulting in reduced reliability.

Therefore, the moving hanger 1000 may be provided such that one driver 1200 generates power to rotate the plurality of power transmission units 1400.

The moving hanger 1000 may preferentially transmit the power generated by the driver 1200 to some of the power transmission units 1400 or some of the reciprocating rotation units 1500 and may secondarily transmit the power to the other power transmission units 1400 or the other reciprocating rotation units 1500.

For example, the reciprocating rotation unit 1500 may be provided to receive power transmitted from the driver 1200 or the displacement generator 1300 and transmit the power to some of the power transmission units 1400. That is, the moving hanger 1000 is provided to centrally transmit the power generated by the driver 1200 to one reciprocating rotation unit 1500, and thus the power transmission structure may be designed simply and power loss may be minimized.

The moving hanger 1000 may be provided to transmit the power transmitted from the driver 1200 to one reciprocating rotation unit 1500 to rotate a specific power transmission unit 1400 connected to the reciprocating rotation unit 1500.

The moving hanger 1000 may further include a connector 1600 provided to transmit power transmitted to a specific power transmission unit 1400 to another power transmission unit 1400.

For example, the connector 1600 may be provided to connect a plurality of power transmission units 1400 to each other. Accordingly, when one power transmission unit 1400 rotates, the connector 1600 may rotate all of the plurality of power transmission units 1400.

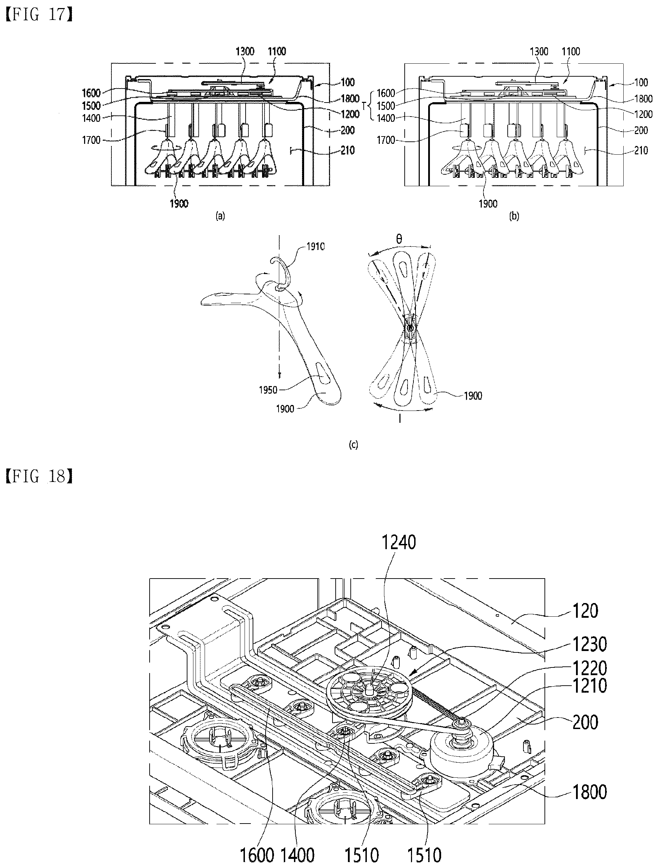

FIG. 17 shows a method of operating a moving hanger.

Referring to FIG. 17(a), the power transmission unit 1400 may rotate to the right by the reciprocating rotation unit 1500 when the driver 1200 operates. In this case, all power transmission units 1400 connected to the connector 1600 may also rotate to the right.

Referring to FIG. 17(b), the power transmission unit 1400 may rotate to the left by the reciprocating rotation unit 1500 when the driver 1200 operates further. In this case, all power transmission units 1400 connected to the connector 1600 may also rotate to the left.

As this process is repeated, the power transmission unit 1400 may rotate left and right.

In this case, the power transmission unit 1400 may be provided to rotate left and right while being fixed in a fixed position. The power transmission unit 1400 may be fixed to the support portion 1800 such that there is no change in position back and forth and left and right when rotating.

The power transmission unit 1400 may be fixed such that a position of the power transmission unit 1400 does not move in vertical, forward, and horizontal directions.

However, the power transmission unit 1400 may be provided to rotate left and right using vertical or height direction in which the power transmission unit extends as a rotation axis. As a result, when the driver 1200 is driven, the hanger 1700 rotates left and right around the power transmission unit 1400 as an axis, and there may be no positional movement.

Referring to FIG. 17(c), the clothes hanger 1900 may include a ring portion 1910 mounted on the hanger 1700 and an accommodation portion 1900 coupled to the ring portion 1910. A surface of the accommodation portion 1950 may include a surface portion 950 that prevents laundry from slipping.

The accommodation portion 1950 may be provided symmetrically left and right with the ring portion 1910 as the center. The clothes hanger 1900 may be mounted on the hanger 1700 such that the accommodation portion 1950 is located in in front and rear directions.

When the power transmission unit 1400 rotates to the left, based on the ring portion 1910, a left side of the accommodation portion 1950 of the clothes hanger 1900 may rotate to the left, and a right side of the accommodation portion 1950 may rotate to the right. In this case, an angle I at which the left side of the seat 1950 rotates may be equal to an angle (theta) at which the right side of the accommodation portion 1950 rotates, and a distance by which the left side of the accommodation portion 1950 moves may be equal to a distance by which the right side of the accommodation portion 1950 moves.

As a result, the weight and force moving to the left with respect to the clothes hanger 1900 and the weight and force moving to the right may be equal to each other, and thus may cancel each other out.

Likewise, even if the power transmission unit 1400 rotates to the right, as a result, the weight and force moving to the left with respect to the clothes hanger 1900 may be equal to each other and cancel each other out.

As a result, even if the power transmission unit 1400 rotates, the forces applied to the hanger unit 1900 may cancel each other out, and as a result, the vibration force, exciting force, or inertial force generated in the clothes hanger 1900 itself may be minimized. As a result, the inertial force generated from the plurality of power transmission units 1400 may be minimized, thereby minimizing vibration or noise generated throughout the moving hanger 1000, and sharply reducing vibration or noise from occurring throughout the clothing treatment apparatus 1.

As a result, even if the driver 1200 rotates at maximum output, vibration occurring in the moving hanger 1000 or the entire clothing treatment apparatus 1 may not be large.

Instead, each of the surfaces of the laundry mounted on the clothes hanger 1900 is shaken by rotating left and right, and thus a large shaking force may be ensured.

As described above, the power transmission unit 1400 may be provided to pass through the inner case 200 and may be provided to receive the power and rotate back and forth clockwise and counterclockwise. As a result, the power transmission unit 1400 may reciprocate left and right at an upper side of the inner case 200 with its position fixed. The power transmission unit 1400 is fixed such that its position does not change up, down, left and right. Not only the upper side of the power transmission unit 1400 but also the lower side is fixed such that the position does not change up, down, left, and right.

That is, the power transmission unit 1400 may be provided to reciprocate at a certain angle in less than one rotation while the rotation center is fixed.

As a result, no matter how fast the power transmission unit 1400 rotates, the position of the clothes hanger 1900 is fixed and one end thereof rotates or moves to one side and the other end moves to the other side, and thus force and vibration transmitted to the power transmission unit 1400 may cancel each other out.

Accordingly, vibration and noise generated by the power transmission unit 1400, the hanger 1700, and the clothes hanger 1900 inside the inner case 200 may be minimized.

As a result, the clothing treatment apparatus may rotate the driver 1200 at a higher rpm to reciprocate the power transmission unit 1400 at a higher frequency. Thus, the clothing treatment apparatus may shake the laundry more strongly.

The moving hanger 1000 may have the power transmission unit 1400 that reciprocates and rotates clockwise or counterclockwise in place. Therefore, as described above, no matter how fast the clothes hanger 900 rotates, vibration or inertial force generated from the clothes hanger 1900 and laundry may cancel each other out when transmitted to the power transmission unit 1400.

As a result, the moving hanger 1000 may not be damaged even if the driver 1200 is raised above a certain RPM, and vibration or noise exceeding a limit value may be blocked from being generated in the clothing treatment apparatus.

Therefore, the clothing treatment apparatus may drive the driver 1200 faster than a clothing treatment apparatus using a conventional moving hanger, and the power transmission unit 1400 may be driven at a higher frequency to shake the laundry more strongly.

As a result, the clothing treatment apparatus may more reliably remove foreign substances on laundry through the moving hanger 1000 and remove wrinkles from the laundry more effectively. Additionally, the clothing treatment apparatus may stimulate the laundry more quickly and expose the laundry to more of the supplied steam.

The clothing treatment apparatus may freely adjust the RPM of the driver 1200, and adjust a drive frequency or a drive cycle of the power transmission unit 1400 according to a course.

FIG. 18 shows the moving hanger 1000 according to an embodiment of the present disclosure.

The moving hanger 1000 may transmit the power of the driver 1200 to only one of the plurality of power transmission units 1400 and transmit the power transmitted to the specific power transmission unit 1400 through the connector 1600 to the other power transmission units 1400.

The displacement generator 1300 or the reciprocating rotation unit 1500 may be provided to intensively transmit the power generated by one driver 1200 to one power transmission unit 1400. The connector 1600 may transmit power transmitted to the specific power transmission unit 1400 to all power transmission units 1400.

The connector 1600 may be provided as a rigid body such that a length thereof does not vary, and may be provided to connect all power transmission units 1400.

Accordingly, all power transmission units 1400 may rotate simultaneously in the same direction and at the same angle when the connector 1600 moves. As a result, the moving hanger 1000 may reciprocate and rotate the plurality of power transmission units 1400 at the same angle at the same time or at the same time with one driver 1200.

The moving hanger 1000 may include the driver 1200 fixed to the upper side of the inner case 200 to provide power to the power transmission unit, the reciprocating rotation units 1500 respectively coupled to the plurality of power transmission units 1400, receiving the power, and rotating such that a rotation direction changes repeatedly, and the connector 1600 provided to connect the plurality of reciprocating rotation unit 1500 to each other.

The connector 1600 may include a link bar that connects the plurality of reciprocating rotation units 1500 and rotates the plurality of reciprocating rotation unit 1500 as one unit.

The connector 1600 may be provided in a single piece.

The connector 1600 may be provided to connect all of the power transmission units 1400.

However, when the connector 1600 is provided to connect the reciprocating rotation units 1500 to each other, the connector 1600 may be installed higher than the support portion 1800, preventing the connector 1600 from being exposed inside the inner case 200.

The connector 1600 may be coupled to either the front side or rear side of the reciprocating rotation unit 1500, and at least one of the displacement generator 1300 and the driver 1200 may be located at one of the others of the front side or the rear side of the reciprocating rotation unit 1500. Accordingly, the connector 1600 may be prevented from interfering with the driver 1200.

The connector 1600 may be provided to reciprocate in a width direction of the inner case 200 and rotate the plurality of reciprocating rotation units 1500.

The driver 1200 may include a motor 1210 that rotates a rotation shaft 1210, the power shaft 1240 that rotates together when the rotation shaft 1210 rotates, and a transmission unit 1230 that connects the power shaft 1240 to the rotation shaft 1210 to transmit a rotational force of the rotation shaft 1210 to the power shaft 1240.

The motor 1210 may be fixed to the upper side of the inner case 200 and may be provided to rotate the rotation shaft 1220. However, the rotation shaft 1220 is provided to rotate at a speed that is excessively higher than an appropriate cycle for reciprocating and rotating the power transmission unit 1400 in the motor 1210. Considering this, when RPM of the rotation shaft is lowered, there is a risk that output of the motor 1210 may not be transmitted to the power transmission unit 1400.

To solve this problem, the transmission unit 1230 may be provided to transmit the output of the rotation shaft 1220 to the power transmission unit 1400 without change while lowering the RPM of the rotation shaft 1220.

The transmission unit 1230 may be connected to the rotation shaft 1220 to rotate and may have a larger diameter than the rotation shaft 1220 to rotate. Accordingly, the transmission unit 1230 may be provided to transmit a torque of the rotation shaft 1220 while rotating slower than the RPM of the rotation shaft 1220.

The power shaft 1240 may be provided to rotate by the transmission unit 1230, may be provided separately from the rotation shaft 1230, and may directly transmit power to the power transmission unit 1400.

The reciprocating rotation unit 1500 may be coupled to the power transmission unit 1400 and be rotatable together with the power transmission unit 1400.

The reciprocating rotation unit 1500 may include a reciprocating lever 1510 that is coupled to the upper side of the power transmission unit 1400 and rotates the power transmission unit 1400.

The reciprocating lever 1510 may have a rib or bar shape, the rotation center of which is coupled to the support shaft 1410.

The reciprocating lever 1510 may be coupled to an upper end of each of the plurality of power transmission units 1400, and some of the reciprocating levers 1510 may be connected to the transmission unit 1230 to receive power of the motor 1210.

The reciprocating lever 1510 may be provided to reciprocate at a certain angle when the transmission unit 1230 is rotated by the motor 1210. The power transmission unit 1400 may be coupled to the rotation center of the reciprocating lever 510 and rotated together with the reciprocating lever 1510.

The plurality of reciprocating levers 1510 may be connected and arranged by the connector 1600.

The connector 1600 may be provided to connect ends of the plurality of reciprocating levers 1510 to each other.

Accordingly, when any one of the plurality of reciprocating levers 1510 rotates, the connector 1600 may move such that the plurality of reciprocating levers 1510 may rotate simultaneously and at the same time.

The power transmission unit 1400 and the reciprocating lever 1510 may be supported on the support portion 1800. The motor 1210 and the transmission unit 1230 may also be supported on the support portion 1800.

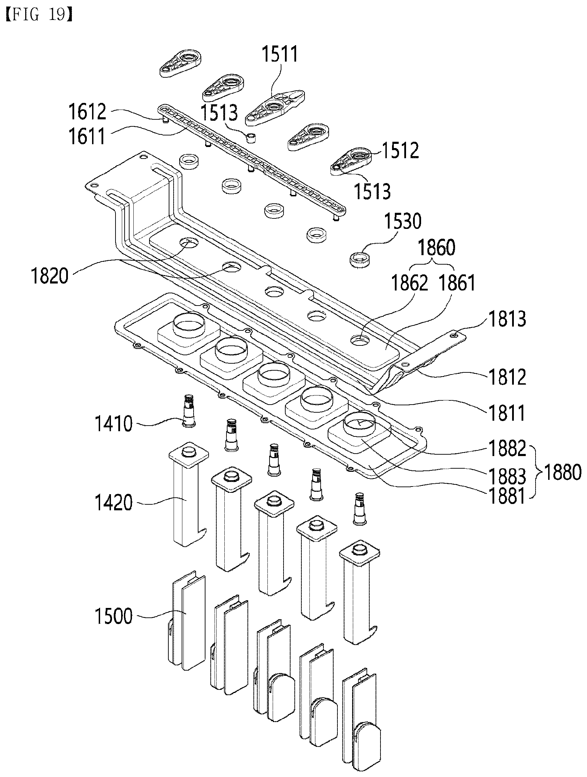

FIG. 19 shows the case in which the moving hanger 1000 is separated from the inner case 200.

The power transmission unit 1400 may be provided extending from the top to the bottom of the inner case, and the hanger 1700 may be coupled to the lower side of the power transmission unit 1400.

The reciprocating rotation unit 1500 may be coupled to each power transmission unit 1400, and may be easily connected to the driver 1200 by being coupled to the upper side of the power transmission unit 1400.

The power transmission unit 1400 and the reciprocating rotation unit 1500 are provided in plural pieces and are arranged at a certain distance apart in the width direction of the inner case.

The connector 1600 is provided to connect the plurality of power transmission units 1400 or the plurality of reciprocating rotation portions 1500 to each other. Thus, the connector 1600 is provided to rotate all of the plurality of power transmission units 1400 or the plurality of reciprocating rotation portions 1500 simultaneously.

The power transmission unit 1400 may include the support shaft 1410 that is formed to pass through the upper side of the inner case 200 and is coupled to the reciprocating lever 1510.

The support shaft 1410 may be formed to pass through the support portion 1800 and may be exposed to the upper side of the support portion 1800 or the upper side of the inner case 200.

The power transmission unit 1400 may include an auxiliary support portion 1420 coupled to the support shaft 1410 and exposed to the accommodation space. The auxiliary support portion 1420 may be provided in a shape of a bar, and the hanger 1700 may be fixedly coupled to a lower side of the auxiliary support portion 1420.

The auxiliary support portion 1420 may be fixed to the support shaft 1410 and rotate together with the support shaft 1410. Accordingly, when the support shaft 1410 rotates by the reciprocating lever 1510, the auxiliary support portion 1420 coupled to the support shaft 1410 also rotates, and thus the hanger 1700 may also rotate left and right.

The reciprocating lever 1510 may include a main lever 1511 that receives power directly from the driver 1200 and reciprocates, and an auxiliary lever 1512 that receives power from the main lever 1511 through the connector 1600.

The main lever 1511 may be provided as a single unit to directly receive power from the driver 1200.

In the driver 1200, the motor 1210 may include a vertical motor 1211 coupled to the support portion 1800 and a vertical rotation shaft 1221 rotated by the vertical motor 1211.

The transmission unit 1230 may include a power pulley 1231 that is coupled to the vertical rotation shaft 1221 and rotates with the vertical rotation shaft 1221, a transmission pulley 1232 that is coupled to the power shaft 1240 to rotate the power shaft 1240, and a belt 1233 connecting the power pulley 1231 to a portion of an outer peripheral surface of the transmission pulley 1232.

The transmission unit 1230 may further include a pulley support portion 1224 that rotatably supports the power shaft 1240 and the transmission pulley 1232. The pulley support portion 1224 may be provided to support the transmission pulley 1232 to be arranged in parallel with the power pulley 1231 and may be accommodated on the support portion 1800.

The power shaft 1240 may be provided to transmit power transmitted from the rotation shaft 1220 to one of both ends of the main lever 1511.

The displacement generator 1300 may be coupled to the power shaft 1240 to receive power. The displacement generator 1300 may be connected to the main lever 1511 and may reciprocate and rotate the main lever 1511 about the support shaft 410.

For example, the displacement generator 1300 may include an eccentric shaft that is eccentrically coupled to the power shaft 1240 and rotates at a constant radius with respect to the rotation center of the power shaft 1240.

The displacement generator 1300 may be connected to an end of the main lever 1511. The displacement generator 1300 may rotate the end of the main lever 1511 around the support shaft 410 while rotating by the power shaft 1240.

The connector 1600 may include a link bar 1610 that connects one end of the main lever 1511 that is not connected to the power shaft 1240 or the displacement generator 1300 to one end of the auxiliary lever 1512.

The auxiliary lever 1512 may be rotatably coupled to the remaining portion of the support shaft 1410, which is not coupled to the main lever 1511 and may extend in one direction from the portion coupled to the support shaft 1410 to be connected to the link bar 1610.

The link bar 1610 may be provided in the form of a straight frame connecting one end of the main lever 1511 and one end of the auxiliary levers 1512. In this case, one end of the main lever 1511 and one end of the auxiliary lever 1512 may be arranged parallel to each other based on the link bar 1610 or the width direction.

The link bar 1610 may be provided in a single piece to rotate the main lever 1511 and the auxiliary lever 1512 simultaneously and simultaneously about the respective support shafts 1410.

The inner case 200 may include the through hole 230 in which a portion of the support portion 1800 is accommodated to expose the power transmission unit 1400 to the receiving space 220.

The through hole 230 may be provided in an upper surface 22 of the inner case, and the through hole 230 may be provided in a direction in which the power transmission units 1400 are arranged.

For example, the power transmission units 1400 may be arranged apart from each other in the width direction of the inner case, and the through holes 230 may be arranged in the width direction of the inner case.

The clothing treatment apparatus 1 may further include a support frame 120 located outside the inner case to support the cabinet 100.

The support frame 120 may be located at each position corresponding to an edge of the cabinet 100 or an edge of the inner case 200 and may include a metal material that maintains the appearance of the clothing treatment apparatus. Both ends of the support portion 1800 are accommodated and supported on the support frame 120, thereby preventing unnecessary shock or load from being transmitted to the upper surface 220 of the inner case.

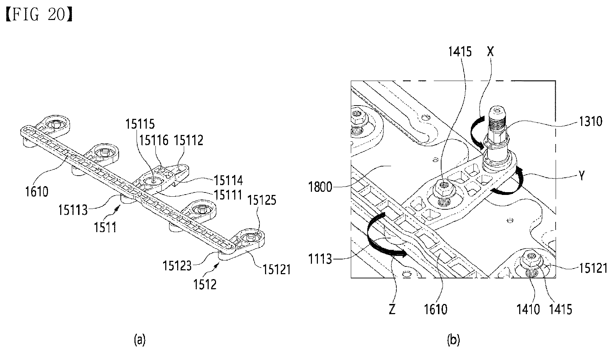

FIG. 20 shows an operating method of the moving hanger 1000.

Referring to FIG. 20(a), the main lever 1511 may include a main body 15111 coupled to the support shaft 1410 and the link bar 1610.

The main body 15111 may include a main center hole 15115 that is coupled to the support shaft 1410 and rotates the support shaft 1410 and extend from the main center hole 15115 to both sides.

The main body 15111 may include a main receiving hole 15112 at one end, which receives power from the driver 1200, and a main transmission hole 15113 at the other end, into which the link bar 1610 is accommodated and coupled.

The main body 15111 may further include a step portion 15114 extending from the center hole to the main receiving hole 15112 and forming a step. In the main body 15111, due to the step portion 15114, one end of the main body 15111 or the main transmission hole 15113 may be located lower than the main center hole 15115.

As a result, a length in which the power shaft 1240 or the eccentric shaft 1310 disposed above the main center hole 15115 extends from the transmission unit 1230 may be ensured.

The auxiliary lever 1512 may include an auxiliary body 15121 including an auxiliary center hole 15125 coupled to the support shaft 1410 and an auxiliary transmission hole 15123 extending to one side from the auxiliary center hole 15125 and coupled to the link bar 1610.

The auxiliary body 15121 may be provided with a shorter length than the main body 15111.

A distance from the main center hole 15115 to the main transmission hole 15113 may be set equal to a distance from the auxiliary central hole 15125 to the auxiliary transmission hole 15123.

The link bar 1610 may be accommodated above the auxiliary transmission hole 15123 and the main transmission hole 15113 to connect the auxiliary lever 1512 and the main lever 1511 to each other.

Referring to FIG. 20(b), the driver 1200 may be provided such that the power shaft 1240 is inserted into the main receiving hole 15112. As a result, the main receiving hole 15112 may be be rotated left and right by directly rotating the power shaft 1240.

In other words, sufficient displacement may not be generated to rotate the main receiving hole 15112 left and right with respect to the main center hole 15115 just by rotating the power shaft 1240.

To this end, the displacement generator 1300 may be coupled to the power shaft 1240 to generate a displacement larger than a rotation radius of the power shaft 1240.

The displacement generator 1300 may be provided to convert an in-place rotational motion of the power shaft 1240 into a displacement motion that moves back and forth over a certain range. The displacement motion is transmitted to the reciprocating rotation unit 1500 such that the power transmission unit 1400 may reciprocate.

As described above, the displacement generator 1300 may include the eccentric shaft 1310 that extends to the power shaft 1240 and rotates along a predetermined radius.

The eccentric shaft 1310 has a smaller diameter than the power shaft 1240 and may be coupled to or extended to the power shaft 1240 at a certain distance from the rotation center of the power shaft 1240.

Accordingly, when the power shaft 1240 rotates, the eccentric shaft 1310 may rotate in a circle, a radius of which is the distance from the power shaft 1240.

A diameter of the eccentric shaft 1310 may be set smaller than a diameter or width of the main receiving hole 15112.

The eccentric shaft 1310 may be inserted and supported in the main receiving hole 15112.

However, a constant radius around which the eccentric shaft 1310 rotates may be set larger than the width or diameter of the main receiving hole 15112. As a result, when the eccentric shaft 1310 rotates, the main receiving hole 15112 may be pushed by the eccentric shaft 1310 and may move left and right based on the main center hole 15115.

As a result, when the eccentric shaft 1310 rotates in a specific direction x, the main receiving hole 15112 of the main body 1511 may also rotate in a specific direction y, and as a result, the center hole 15115 of the main body may also rotate in the same direction as the main receiving hole 15112, and the main transmission hole 15113 may reciprocate and rotate in a direction z opposite to the constant direction.

When the eccentric shaft 1310 rotates, the support shaft 1410 may reciprocate and rotate together with the main center hole 15115 such that the power transmission unit 1400 may reciprocate, and the main transmission hole 15113 may also reciprocate and rotate such that the link bar 1610 may reciprocate and rotate, and accordingly, the auxiliary lever 1521 may also reciprocate and rotate around the auxiliary center hole 15125 and the support shaft 1410. The power transmission unit 1400 coupled to the auxiliary lever 1521 may also reciprocate and rotate.

The power transmission unit 1400 may be provided with a screw thread along a circumference of an upper side of the support shaft 1410.

The main transmission hole 15113 and the auxiliary center hole 15125 may be directly coupled and fixed to the support shaft 1410 by using the thread or the like.

However, in the power transmission unit 1400, the support shaft 1410 may further include a transmission coupler 1415 that passes through the main transmission hole 15113 and the auxiliary center hole 15125 without change and then couples the support shaft 1410 to the screw thread of the support shaft 1410 to fix the main transmission hole 15113 and the auxiliary center hole 15125.

Therefore, the support shaft 1410 and the reciprocating lever 1510 are coupled to each other due to the transmission coupler 1415, and thus the support shaft 1410 and the reciprocating lever 1510 may rotate simultaneously.

Hereinafter, an embodiment in which the controller 700 of the clothing treatment apparatus performs an arbitrary course will be described based on the above configuration.

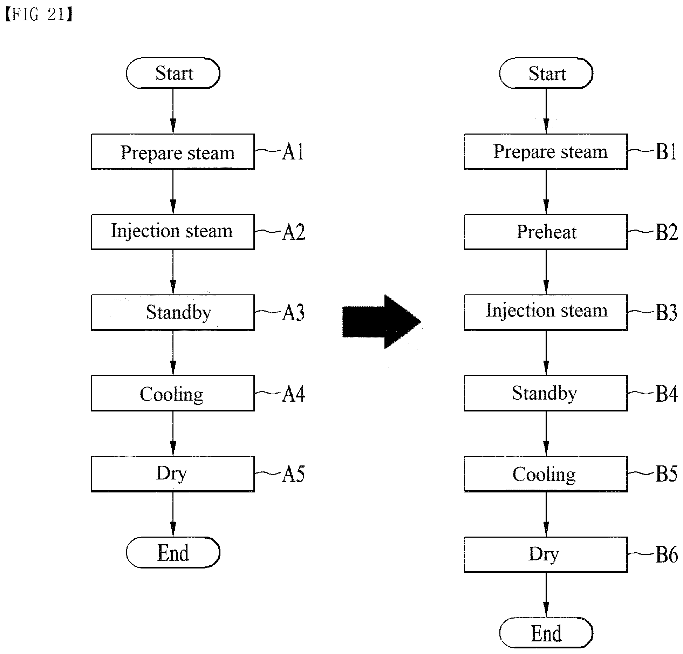

FIG. 21 shows a process in which a clothing treatment apparatus performs a laundry treating course.

The clothing treatment apparatus may set allowable electrical energy as the maximum electrical energy to be used. The controller 700 of the clothing treatment apparatus may be set to block power supply when detecting that more than the allowable electrical energy is consumed.

The allowable electrical energy may be less than electrical energy consumed when the first heater 841, the second heater 842, and the compressor 342 are driven simultaneously. As a result, the clothing treatment apparatus is set such that the entire steam heater 840 and the compressor 342 may not be driven simultaneously.

As a result, the clothing treatment apparatus may be set not to drive the first heater 841 and the second heater 842 simultaneously while the compressor 342 is driven and not to drive the compressor 342 while the first heater 841 and the second heater 842 are driven simultaneously.

Therefore, the clothing treatment apparatus may be set such that it is impossible to supply the maximum amount of steam to be supplied while hot air is supplied inside the inner case.

However, the allowable electrical energy may be greater than electrical energy consumed when the first heater 841 and the second heater 842 are driven simultaneously. The allowable electrical energy may be greater than electrical energy consumed when the first heater 841 and the compressor 342 are driven simultaneously. The allowable electrical energy may be greater than electrical energy consumed when the second heater 842 and the compressor 342 are driven simultaneously.

As a result, the first heater 841 and the second heater 842 may be driven simultaneously, the first heater 841 and the compressor 342 may be driven simultaneously, and the second heater and the compressor 342 may be driven simultaneously.

Therefore, in the clothing treatment apparatus, when the compressor 342 is not driven, the first heater 841 and the second heater 842 may be driven simultaneously, thereby supplying the maximum amount of steam to be supplied inside the inner case.

In the clothing treatment apparatus, one of the first heater 841 and the second heater 842 may be driven simultaneously with the compressor 342, hot air may be simultaneously supplied into the inner case 200 while the steam is supplied into the inner case 200, and steam may be simultaneously supplied into the inner case 200 while hot air is supplied.

The clothing treatment apparatus may initially drive the first heater 841 and the second heater 842 simultaneously to rapidly heat water inside the steam generator 810 to generate steam first. Then, when steam is generated, the clothing treatment apparatus may drive only one of the first heater 841 and the second heater 842 to maintain the temperature of water at a boiling point to continue generating steam.

However, in this case, after steam is generated, the amount of steam sprayed may be reduced compared to when the first heater 841 and the second heater 842 are driven simultaneously.

The clothing treatment apparatus may adjust a steam supply portion amount by driving only one of the first heater 841 and the second heater 842. Therefore, a sufficient refresh cycle may be performed by supplying only a small amount of steam to materials that are vulnerable to moisture and temperature, such as silk or cashmere.

When supplying steam into the inner case, the clothing treatment apparatus may use only one of the first heater 841 and the second heater 842, thereby reducing a power consumption required for steam supply portion.

In addition, the clothing treatment apparatus may quickly increase the temperature inside the inner case by driving the compressor 342 together when supplying steam. As a result, the clothing treatment apparatus may quickly complete a drying cycle to remove moisture from wet laundry, and a refresh cycle to sterilize, deodorize, and remove wrinkles from laundry.

FIG. 21(a) shows an embodiment of a method of controlling a clothing treatment apparatus.

When performing a refresh course or a standard course for performing the refresh cycle, the clothing treatment apparatus may perform a steam preparation operation A1 of heating water by driving the steam heater, and a steam injection operation A2 of supplying the steam to laundry when water is heated to generate water while the steam preparation operation A1 is performed.

In general, an object of the refresh cycle may be to supply moisture to laundry in a dry state, and thus a large amount of steam injected to the laundry is required.

Therefore, the clothing treatment apparatus may simultaneously drive the first heater 841 and the second heater 842 in both the steam preparation operation A1 and the steam injection operation A2.

In the steam preparation operation A1 and the steam injection operation A2, the compressor 342 may not be driven in consideration of the allowable electrical energy.

The clothing treatment apparatus may perform a standby operation A3 of providing a time for the laundry to be moistened with supplied steam.

When the standby operation A3 is performed for a certain time, the clothing treatment apparatus may perform a cooling operation A4 of driving only a blowing fan by further lowering a surface temperature of laundry before the compressor is driven. Thermal damage to laundry may be prevented through the cooling operation A4.

When the internal temperature of the inner case 200 decreases and the moisture content of the laundry is sufficient, the clothing treatment apparatus may perform a drying operation A5 of driving one or more of the compressor 342 and a blowing fan 351.

Through the drying operation A5, a refresh cycle including deodorizing and removing wrinkles from laundry may be performed while drying the moisture contained in the laundry. The drying operation A5 may have the longest duration compared to all previously performed operations.

In the steam injection operation A2, the compressor is not driven, and thus hot air may not be supplied. Therefore, until reaching the drying operation A5, the internal temperature of the inner case 200 and the temperature of a refrigerant flowing through the heat supply portion 400 are relatively low temperatures.

Therefore, in the above-described control method, the drying operation A5 needs to last relatively long to ensure refresh performance, including any one of complete drying, sterilization, deodorization, and wrinkle removal of laundry.

FIG. 21(b) shows another embodiment of a method of controlling a clothing treatment apparatus.

However, as described above, the clothing treatment apparatus may have a plurality of separate heaters, and thus some heaters and a compressor may be driven at the same time.

For example, the steam heater 840 includes the first heater 841 and the second heater 842, and thus either one of the first heater 841 and the second heater 842 and the compressor may be driven simultaneously. Based on this, the refresh cycle may be performed in a different manner.

Therefore, the clothing treatment apparatus may quickly increase the temperature inside the inner case 200 before the drying operation by simultaneously driving the heater and compressor before the drying operation, thereby more quickly ensuring the temperature and time required for completely drying, sterilizing, deodorizing, and removing wrinkles from the laundry.

As a result, the duration of the drying operation may be reduced compared to before. The duration of the drying operation may be set to be the longest in the refresh cycle. By reducing the duration of the drying operation, the duration of the entire refresh cycle may be greatly reduced. Energy efficiency may be greatly increased by reducing a driving time of the compressor.

The clothing treatment apparatus may perform a steam preparation operation B1 of driving all of the steam heaters 840 to supply steam to the laundry when any course for refreshing or managing the laundry is performed.

In the steam preparation operation B1, a time of the refresh cycle may be reduced as the steam is generated faster, and thus the controller 700 may drive all heaters of the steam heater 840.

In other words, in the steam preparation operation B1, the controller 700 may heat water by using the maximum power of the steam heater 840.

When the steam heater 840 includes the first heater 841 and the second heater 842, the first heater 841 and the second heater 842 may be driven simultaneously to heat water.

When detecting that steam is generated inside the steam case 810 through a detection device 860 in the steam preparation operation B1, the clothing treatment apparatus may perform a preheating operation B2 of driving only some of the steam heater 840.

That is, after steam is generated in the steam case 810, even if only one of the first heater 841 and the second heater 842 is driven, the steam may be continuously generated and may be supplied to the inner case 200.

In the preheating operation B2, the controller 700 controls only one of the first heater 841 and the second heater 842 to supply the steam to the inner case 200.

Accordingly, the clothing treatment apparatus may increase the moisture content of the laundry by increasing the temperature inside the inner case 200 by using the steam from the preheating operation B2. Only a portion of the steam heater 840 is used in the preheating operation B2, and thus extra electrical energy may be ensured.

In the preheating operation B2, the clothing treatment apparatus may drive one or more of the compressor 342 and the blowing fan 351. As a result, hot air may also be supplied into the inner case 200.

As a result, in at least some sections in the preheating operation B2, the compressor 342, the blowing fan 351, and a portion of the steam heater 840 may overlap each other and operate together.

In the preheating operation B2, either the first heater 841 or the second heater 842 and the compressor 342 may be driven simultaneously. Steam and hot air may be supplied simultaneously inside the inner case 200.

When either the first heater 841 or the second heater 842 and the compressor 342 are driven simultaneously, the temperature inside the inner case 200 may be increased from the beginning of the refresh cycle, and higher-temperature air may be introduced into the evaporator 341. As a result, the heat exchange performance of the evaporator 341 may be enhanced to heat the condenser 343 to a higher temperature. Accordingly, hot air of a higher temperature may be supplied into the inner case 200, and thus the inside of the inner case 200 may reach the optimal or minimum temperature required for the refresh cycle more quickly. Therefore, a time to perform a subsequent refresh process may be greatly shortened.

The overall coefficient of performance (cop) of the heat supply portion 340 may also be increased.

The preheating operation B2 may include one or more of a section in which either the first heater 841 or the second heater 842 and the compressor 342 are driven simultaneously and a section in which only the compressor 342 is driven.

For example, in the preheating operation B2, there may not only be a section in which the steam heater 840 and the compressor 342 are simultaneously driven, but there may also be a section in which only the compressor 342 is driven.

However, the section in which only the compressor 342 is driven may be placed after the section in which the steam heater 840 and the compressor 342 are driven.

This is because it is more advantageous to drive the heat supply portion 340 when air with high heat capacity is supplied to the evaporator 341 before driving the compressor 342. That is, when the steam is supplied to the inner case 200 through the steam heater 840, not only does the temperature inside the inner case 200 rise, but an absolute humidity also increases, creating air with a high heat capacity to come into contact with the evaporator 341.

In detail, the preheating operation B2 may include a first section of driving either the first heater 841 or the second heater 842 and the compressor 342, and a second section of stopping driving of the first heater 841 and the second heater 842 and driving only the compressor 342.

Hot air and steam may be supplied to the inner case 200 through the first section to increase the temperature inside the inner case 200 and the amount of heat of the air flowing into the circulation duct 320.

Through the second section, hot air may be fully supplied to the inside of the inner case 200 to satisfy a minimum temperature condition for refreshing laundry such as deodorization, wrinkle removal, and sterilization.

In the preheating section B2, the first heater 841 may be driven. As a result, a larger amount of steam may be supplied into the inner case 200 than when driving the first heater 841, thereby further increasing the temperature inside the inner case 200 and the amount of heat of air flowing into the circulation duct 320.

In the preheating section B2, the second heater 841 may be driven.

Accordingly, in the preheating section B2, the temperature inside the inner case 200 may be gradually increased and the increased temperature may be maintained more stably for a longer time. As a result, a time required for sterilization or the like may be ensured.

The laundry may be prevented from being deformed due to high humidity.

The clothing treatment apparatus may be set to stop driving of the steam heater 840 when the temperature inside the inner case 200 rapidly rises or the temperature of a refrigerant discharged from the compressor 342 exceeds a limit temperature in the preheating section B2.

The clothing treatment apparatus may be set such that either the first heater 841 or the second heater 842 is repeatedly driven and stopped (ON/OFF) in the preheating section B2. That is, when the temperature of the refrigerant approaches or exceeds the limit temperature, the controller 700 may stop driving the steam heater 840, and when the temperature of the refrigerant reaches a safe temperature lower than the limit temperature, the controller 700 may repeatedly redrive the steam heater 840. When the internal temperature of the inner case approaches or exceeds a target temperature, the controller 700 may stop driving of the steam heater 840, and when the internal temperature reaches a cooling temperature lower than the target temperature, the controller 700 may repeatedly redrive the steam heater 840.

Even if the steam heater 840 is driven intermittently, an object of the preheating section B2 is to quickly increase the internal temperature of the inner case 200, and thus a time to drive the first heater 841 and the second heater 842 may be set to be longer than a time to stop the first heater 841 and the second heater 842.

The preheating section B2 may be performed for a predetermined time and may be terminated when the internal temperature of the inner case or the temperature of the refrigerant maintains a predetermined temperature for more than a predetermined time.

When the preheating section B2 is completed, the clothing treatment apparatus may perform a steam injection operation B3 of fully supplying steam to the inside of the inner case 200. In the steam injection operation B3, the first heater 841 and the second heater 842 may be driven simultaneously to supply a large amount of steam to the inner case 200.

The steam injection operation B3 is performed while the laundry placed in the inner case 200 is heated to a certain level in the preheating operation B2, and thus the moisture content of the entire laundry may be greatly increased.

The steam injection operation B3 may be performed shorter than the steam injection operation A2 without previously performing the preheating operation B2. This is because a certain level of steam is already supplied in the preheating operation B2, and thus the amount of steam to be additionally supplied is set to be small.

The steam injection operation B3 may be terminated when the ensured time for sufficiently moistening the laundry elapses.

When the steam injection operation B3 is terminated, a drying operation B6 of drying the laundry with hot air may be performed. However, when the steam injection operation B3 is terminated, a standby operation B4 of waiting for a certain time to ensure a time for steam injected into the inner case 200 to the entire laundry may be additionally performed.

The standby operation B4 may correspond to a state in which the steam heater 840 and the compressor 342 are not driven.

Through the standby operation B4, it may be possible to prevent the steam injected into the inner case from evaporating immediately before being moistened by the laundry and prevent the laundry from being damaged due to rapid increase of a surface temperature of the laundry by further applying hot air in a state in which the surface temperature of the laundry rises due to steam.

The drying operation B6 is an operation of fully supplying hot air to the inner case 200 by driving the compressor 342. Therefore, when hot air is supplied immediately while the temperature inside the inner case 200 does not fall below the safe temperature, the temperature inside the inner case 200 may rise above the limit temperature to damage the laundry.

Therefore, a cooling operation B5 of driving the blowing fan 352 may be performed before performing the drying operation B6. In the cooling operation B5, the controller 700 may drive the blowing fan 352 but block the compressor 342 and the steam heater 340 from being driven.

In the cooling operation B5, air inside the inner case 200 may be cooled by circulating along a circulation duct 200.

When the temperature inside the inner case 200 falls below the safe temperature or the cooling operation B5 is performed for a required time, the cooling operation B5 may be terminated.

The clothing treatment apparatus may perform the drying operation B6 of supplying hot air to the laundry. The drying operation B6 may be performed until the moisture content of the laundry falls below a certain level.

The controller 700 may recognize that the moisture content is reduced below a certain level by detecting the temperature inside the inner case 200 or the temperature of the refrigerant.

For example, when the moisture content of laundry is high, even if the drying operation B6 is performed, the temperature flowing back into the circulation duct 320 due to the heat of vaporization may not rise, or may not rise rapidly even if the temperature rises.

However, when most of the laundry is dried and the moisture content is below a certain level, the heat of vaporization is small, and thus the temperature flowing into the circulation duct 320 may rise rapidly, and may ultimately correspond to the temperature of hot air.

This allows the temperature of the refrigerant to move in a similar pattern to the temperature flowing into the circulation duct 320.

When the controller 700 detects that the moisture content of the laundry is lowered, sufficient drying of the laundry is obtained, and when the minimum time required for refreshing elapses, the controller 700 may terminate the drying operation B6.

Alternatively, the controller 700 may terminate the drying operation B6 when a time during which the laundry is exposed to the minimum drying temperature condition or more reaches the minimum time.

As a result, the clothing treatment apparatus may further arrange the preheating operation B2 before the drying operation B6, thereby increasing the total time for the temperature inside the inner case 200 to reach the minimum condition temperature or higher, and thus the duration of the drying operation B6 may be greatly reduced.

The clothing treatment apparatus may continuously maintain a state in which driving of the compressor 342 is stopped until the drying operation B6 is performed after the compressor 342 is driven and stopped in the preheating section B2.

Hereinafter, an embodiment of optimally determining the capacity of heaters when a plurality of steam heaters 840 are provided will be described.

As described above, the controller 700 is provided to control the heat supply portion 340 and the steam supply portion 800 below the allowable electrical energy to perform an arbitrary course for processing the laundry.

The total of driving power of the compressor 342, driving power of the first heater 841, and driving power of the second heater 842 may be set to be greater than the allowable electrical energy.

When the sum of the driving power of the compressor 342 and the driving power of the first heater 841 is set to be less than the allowable electrical energy, and the sum of the driving power of the compressor 342 and the driving power of the second heater 841 is set to be less than the allowable electrical energy, the specifications of the compressor 342, the first heater 841, and the second heater 842 may be set in various ways.

The driving power of the compressor 342 may be defined as electrical energy required when the compressor 342 is driven at maximum rpm, and may be determined as one of the individual specifications of the compressor 342.

The driving power of the first heater 841 may be defined as the electrical energy required to drive the first heater 841 and may be determined as one of the individual specifications of the first heater 841.

The driving power of the second heater 842 may be defined as the electrical energy required to drive the second heater 842 and may be determined as one of the individual specifications of the second heater 842.

The driving power of the compressor 342 may be determined as electrical energy for removing all moisture contained in the laundry within an allowable time when the controller 700 performs the arbitrary course.

The driving power of the second heater 842 is set to be less than the driving power of the first heater 841. A reference for determining the driving power of the first heater 841 and a minimum reference for determining the driving power of the second heater 842 may be necessary.

There is no problem in driving the first heater 841 or the second heater 842 with any driving power as long as the driving power of the first heater 841 or the second heater 842 is equal to or less than a value obtained by subtracting the driving power of the compressor from the allowable electrical energy.

However, when the driving power of the first heater 841 or the second heater 842 is set to be excessively low, it may be impossible to supply the heat and moisture required to perform the above arbitrary course into the inner case 200.

When the driving power of the first heater 841 or the second heater 842 is set to be excessively high, the heat and moisture required to perform the above arbitrary course may be excessively supplied to the inner case 200.

The driving power of the second heater 842 is set to be lower than that of the first heater 841, and thus it may be necessary to first determine the optimal driving power of the second heater 842.

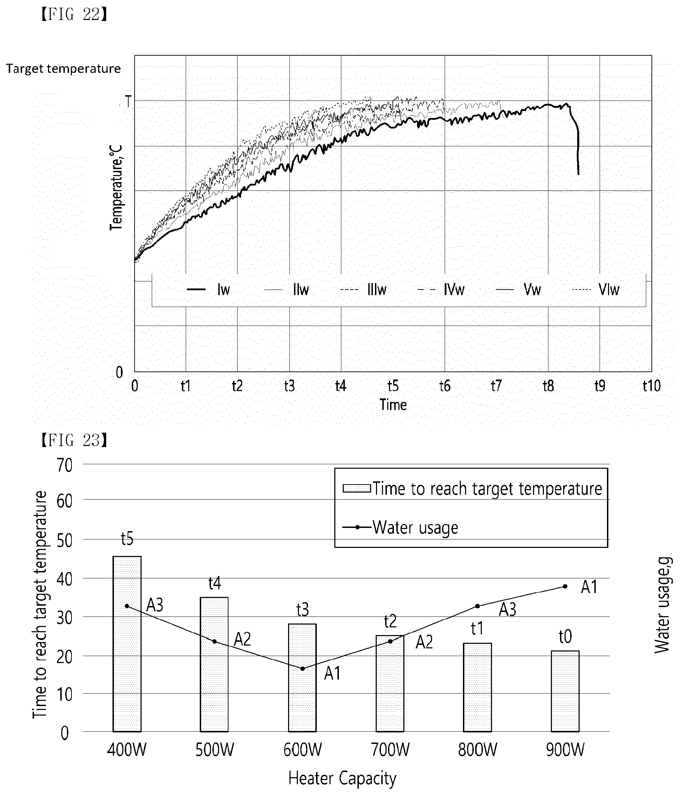

FIGS. 22 and 23 show an embodiment in which the specifications of the steam heater are determined based on temperature and water usage. Referring to FIG. 22, a time required to reach a specific temperature when the steam heater is driven with various specifications is summarized.

The controller 700 uses the steam heater 840 to increase the moisture content of laundry and increase the internal temperature of the inner case 200 in a process of performing a certain course. However, the inner case 200 is sealed, and thus the moisture content of the laundry may be sufficiently increased during the course no matter what driving power the steam heater 840 is provided. Therefore, the steam heater 840 needs to ensure the minimum driving power for raising the temperature inside the inner case 200 above a set temperature.

The set temperature may be defined as the minimum temperature for removing creases and wrinkles from laundry, and a target temperature T may be a temperature higher than the set temperature and may be defined as a temperature for eradicating bacteria and mites from the laundry while evaporating all moisture contained in the laundry.

For example, the set temperature may be defined as 37 degrees or higher, and the target temperature T may be defined as 60 degrees or higher.

The driving power of the second heater 842 may be set to a specification that may increase the internal temperature of the inner case 200 to at least the target temperature T or higher.

Even if the internal temperature of the inner case 200 increases to the target temperature T by driving the second heater 842, when the target temperature is reached excessively late, excessively much time may elapse and energy efficiency may be reduced when performing the course.

Thus, the driving power of the second heater 842 may be set to a specification that may increase the internal temperature of the inner case 200 to the target temperature T or higher before a delay time t7. The delay time may be determined as an average time that the steam heater 840 is driven during the arbitrary course.

For example, the delay time t7 may be set to within 40 minutes. When the allowable electrical energy of the clothing treatment apparatus is set to 1500 w to 1600 w, the driving power of the second heater 842 may be determined in the range of 400 w to 900 w in consideration of the driving power of the first heater 841 and the driving power of the compressor 342. FIG. 22 shows a case in which the temperature inside the inner case 200 increases over time when the second heater 842 is provided to have a specification to be driven by a first power I W, is provided to have a specification to be driven by a second power II W higher than the first power, is provided to have a specification to be driven by a third power III W higher than the second power, is provided to have a specification to be driven by a fourth power IV W higher than the third power, is provided to have a specification to be driven by a fifth power V W higher than the fourth power, or is provided to have a specification to be driven by a sixth power VI W higher than the fourth power.

Even if the second heater 842 is provided to have a specification to be driven by any of the first power I W to the sixth power VI W, the internal temperature of the inner case 200 may be seen to reach the target temperature T.

However, it may be seen that, when the second heater 842 is provided to have a specification to be driven by the first power I W, the delay time is exceeded, the temperature inside the inner case 200 reaches the target temperature and that the temperature inside the inner case 200 reaches the target temperature T within the delay time t7 from a time the second heater 842 is provided beyond a specification to be driven by the second power II W.

Therefore, it may be seen that it is desirable that the second heater 842 may be provided to have a specification to be driven by at least the second power II W or higher.

FIG. 23 shows a time taken to reach a specific temperature and the amount of water used when the second heater 842 is driven with various specifications.

It may be seen that, as the driving power of the second heater 842 increases, the time to reach the target temperature decreases. For example, when the driving power of the second heater 842 is set to the first power I W, the longest time may be taken to reach the target temperature.

For example, when the driving power of the second heater 842 is determined with a specification of the first power I W, an extra time t5 may be consumed.

For example, the first power I W may correspond to 400 W.

The extra time t5 may be set to 40 minutes or more, for example, 46 minutes.

The extra time t5 may be longer than the delay time t4 and may be a time that causes a delay in the course.

When the driving power of the second heater 842 is set to the second power II W, it may take a delay time t4 shorter than the extra time t5 to reach the target temperature.

For example, the second power II W may correspond to 500 W.

The delay time t4 may be set within 40 minutes, for example, 35 minutes.

Thus, since the driving power of the second heater 842 is the second power II W, no course delay occurs, and thus it may be considered that the course enters an appropriate specification range. When the driving power of the second heater 842 is set to the third power III W, it may take the delay time t4 and a short safe time t3 to reach the target temperature.

For example, the third power III W may correspond to 600 W.

The safe time t3 may be set within 40 minutes, for example, 28 minutes.

When the driving power of the second heater 842 is set to the fourth power IV W, it may take an economic time t2 shorter than the safe time t3 to reach the target temperature.

For example, the fourth power IV W may correspond to 700 W.

The economic time t2 may be set within 40 minutes, for example, 25 minutes. When the driving power of the second heater 842 is set to the fifth power V W, it may take a quick time t1 shorter than the economic time t2 to reach the target temperature.

For example, the fifth power V W may correspond to 800 W.

The quick time t1 may be set within 40 minutes, for example, 23 minutes. When the driving power of the second heater 842 is set to the sixth VI W, it may take a shortened time t0 shorter than the quick time t1 to reach the target temperature.

For example, the fifth power V W may correspond to 800 W.

The shortened time t0 may be set within 40 minutes, for example, 21 minutes.

It may be seen that, as the driving power specification of the second heater 842 increases, the time to reach the target temperature decreases.

However, a time to reach the target temperature T may not be shortened in proportion to the amount by which the driving power specification of the second heater 842 increases. For example, a difference between the delay time T4 and the safe time T3 may be less than a difference between the extra time T5 and the delay time T4.

A difference between the safe time T3 and the economic time T2 may be less than a difference between the delay time T4 and the safe time T3.

A difference between the economic time T2 and the quick time T1 may be less than a difference between the safe time T3 and the economic time T2.

As such, it may be expected that as the driving power of the second heater 842 increases, a time to reach the target temperature decreases and water usage decreases accordingly, but a time to reach the target temperature is proportional to the difference in driving power and is not shortened, and the water usage may not continue to decrease.

Basically, no matter what driving power the second heater 842 is driven, the second heater 842 needs to be driven for more than 20 minutes to reach the target temperature T.

In other words, as the driving power of the second heater 842 increases, the target temperature is reached more quickly, and thus the water usage tends to decrease. However, as the driving power of the second heater 842 increases, the amount of steam generated per unit time increases, and the driving time is maintained for more than a certain time, and thus a greater amount of steam may be supplied into the inner case 200. Therefore, it may not be concluded that as the driving power of the second heater 842 increases, a smaller amount of water is needed to raise the temperature inside the inner case 200 to the target temperature.

In summary, the second heater 842 needs to be set to specifications for increasing the temperature inside the inner case 200 to the target temperature while using water below an allowable value. The allowable value may be set to the amount of water for performing at least 5 courses when the water supply tank 30 is filled with water above a full level. For example, the allowable value may be set to 440 g or less.

Referring to FIG. 23, as the driving power of the second heater 842 is set higher, such as the first power I W, the second power II W, and the third power III W, the water usage may gradually decrease to an excessive amount A3, a moderate amount A2, and a small amount A1. This may be interpreted as if as the driving power of the second heater 842 increases, a greater amount of steam is supplied into the inner case 200, and thus the internal temperature of the inner case 200 is increased more quickly, thereby reducing the overall water usage.

The small amount A1 and the moderate amount A2 may be defined as the water usage used to ensure that the course is performed more than a target number of times, considering the volume of the water supply tank. For example, the target number of times may be defined as 4 or more. However, when the driving power of the second heater 842 is set to the fourth power IV W, the fifth power V W, and the sixth power VI W that are greater than the third power III W, the water usage may gradually increase to the moderate amount A2, the excessive amount A3, and a super excessive amount A4.

For example, the excessive amount A3 may be set to 460 g, the moderate amount A2 may be set to 430 to 440 g, and an appropriate amount A1 may be set to 420 g.

This may be interpreted as if, as the driving power of the second heater 842 is set to be high, a greater amount of steam may be supplied inside the inner case 200 to reach the target temperature more quickly, but the amount of steam generated per unit time becomes excessively large, and thus water usage increases rather.

Therefore, it may be optimal for the second heater 842 to be set to a driving power between the second power II W and the fourth power IV W, taking comprehensive consideration of the time to reach the target temperature and water usage.

In other words, the specification of the second heater 842 may be determined to have optimal driving power for minimizing water usage while reaching the target temperature as short as possible.

The optimal driving power of the second heater 842, which may save energy while preventing delays in the course performed while using as little water as possible, may be considered to be the third power III V. However, in comprehensive consideration of an environment in which the clothing treatment apparatus is located, the amount of electrical energy supplied, the weight and material of the laundry placed inside the inner case 200, and the like, the optimal driving power of the second heater 842 may be determined in a range of first to fourth power.

The optimal driving power of the first heater 841 may be determined in a range of the second to fourth power by considering the same reference as that of the second heater 842.

In contrast, the driving power of the first heater 841 may be determined to be equal to or less than a value obtained by subtracting the driving power of the second heater from the allowable electrical energy.

For example, the driving power of the first heater 841 may be set to seventh power set higher than the sixth power or eighth power set higher than the seventh power.

For example, the driving power of the first heater 841 may be set in the range of 800 w to 1100 w.

For example, when the allowable electrical energy is 1500 w and the driving power of the second heater 842 is determined to be 600 w, the driving power of the first heater 841 may be determined to be 900 w. The driving power of the first heater 841 may be determined to be 880 w with an error of about 20 w not to exceed the allowable electrical energy when driving the second heater 842 together.

The specification of the steam heater 840 of the clothing treatment apparatus may be determined based on other references, unlike the water usage and temperature conditions described above.

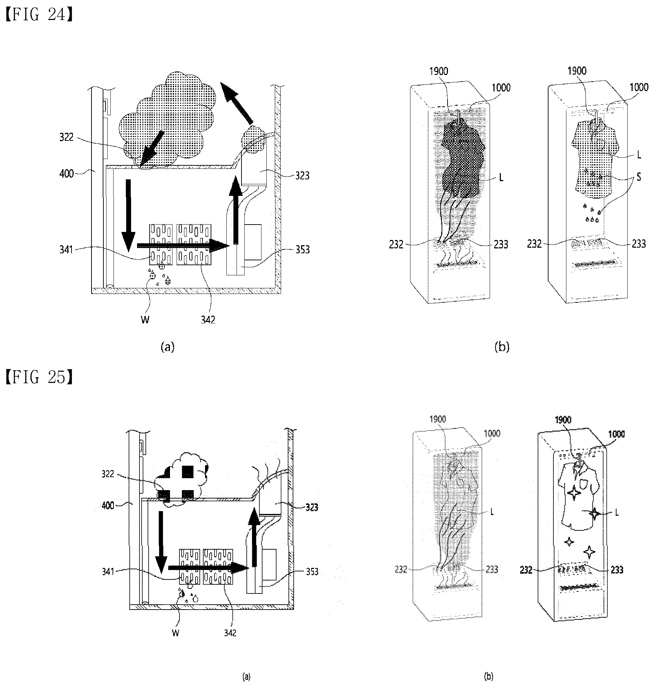

FIGS. 24 and 25 show an embodiment in which the specification of the steam heater is determined based on a dehumidification amount of a heat supply portion.

The clothing treatment apparatus may determine the specification of the steam heater 840 based on the dehumidification performance or dehumidification amount of the evaporator 341.

The main object of the clothing treatment apparatus is not to perform a drying cycle to remove moisture contained in the laundry, but to perform a refresh cycle such as deodorizing, removing wrinkles, and sterilizing the laundry.

The refresh cycle may be performed by supplying moisture to laundry with a low moisture content to increase the moisture content, and then removing foreign substances, odors, and dust on the laundry as the moisture evaporates, and correcting the arrangement of fibers of the laundry to remove wrinkles.

Therefore, in the clothing treatment apparatus, the driving power of the first heater 841 and the second heater 842 needs to be set to generate a greater amount of steam than a dehumidification amount of the heat supply portion 340.

Therefore, in the clothing treatment apparatus, the specification of the first heater 841 and the second heater 842 may be determined such that the sum of the amount of steam generated by the first heater 841 and the amount of steam generated by the second heater 842 is greater than the dehumidification amount to be condensed in the evaporator 341.

As a result, it may be ensured that the internal humidity of the inner case 200 and the moisture content of the laundry are always increased when the first heater 841 and the second heater 842 are driven.

In the clothing treatment apparatus, the first heater 841 may be used to increase not only the temperature inside the inner case 200 but also a relative humidity, and the second heater 842 may be used to maintain or even reduce the relative humidity of the inner case 200, but may be used to intensively increase the temperature inside the inner case 200.

Accordingly, one or more of the second heater 842 and the compressor 342 may be driven in a sterilization course in which maintaining temperature inside the inner case 200 is important and excessive moisture supply is not required, and one or more of the first heater 841 and the compressor 342 may be driven in a standard course or a refresh course for increasing the moisture content of laundry while increasing the temperature inside the inner case 200.

Referring to FIG. 24(a), the first heater 841 may be provided such that the amount of steam generated is greater than the dehumidification amount condensed in the evaporator 341.

In detail, the first heater 841 may be provided such that an average steam generation amount per minute is greater than an average dehumidification amount per minute of the evaporator.

Alternatively, the maximum amount of steam to be generated by the first heater 841 may be greater than the dehumidification amount of the evaporator for condensing moisture to the maximum.

When the first heater 841 and the compressor 342 are driven simultaneously, steam is generated from the first heater 841 and flows into the inner case 200, and the evaporator 341 condenses moisture from air flowing from the inner case 200.

However, the steam injection amount of the first heater 841 is greater than the dehumidification amount of the evaporator 341, and thus some of the steam or moisture may be discharged from the exhaust hole 232.

Referring to FIG. 24(b), the first heater 841 and the compressor 342 may be driven simultaneously to supply hot air and steam into the inner case 200. Then, when driving of the first heater 841 and the compressor 342 is stopped, moisture S may remain inside the inner case 200, and the moisture content of laundry L may be increased.

As a result, when the compressor 342 and the first heater 841 are driven simultaneously for more than a certain time, the humidity inside the inner case may increase.

Even if the compressor 342, the first heater 841, and the second heater 842 are driven simultaneously for more than a certain time, the humidity inside the inner case may increase.

To this end, the driving power of the first heater 841 may be determined in the range of 800 w to 1100 w.

Referring to FIG. 25(a), the second heater 842 may be provided such that the amount of steam generated is equal to or less than the dehumidification amount of the evaporator.

In detail, the second heater 842 may be provided such that an average steam generation amount per minute is equal to or less than an average dehumidification amount per minute of the evaporator.

The second heater 842 may be provided such that the maximum stream generation amount is equal to or less than the maximum dehumidification amount of the evaporator.

The average steam generation amount of the second heater 842 may be set to be less than the average dehumidification amount of the evaporator 341.

When the second heater 842 and the compressor 342 are driven simultaneously, steam is generated from the first heater 842 and flows into the inner case 200, and the evaporator 341 condenses moisture from air flowing from the inner case 200.

However, the steam injection amount of the second heater 842 is greater than the dehumidification amount of the evaporator 341, and thus only hot air from which moisture is removed may be discharged from the exhaust hole 232.

Referring to FIG. 25(b), the second heater 842 and the compressor 342 may be driven simultaneously to supply hot air and steam into the inner case 200. Then, when driving of the second heater 842 and the compressor 342 is stopped, the inside of the inner case 200 may be dried and moisture S may not remain. The laundry L may have a reduced moisture content or may be at least partially dry.

As a result, when the compressor 342 and the second heater 842 are driven simultaneously for more than a certain time, the humidity inside the inner case 200 may be maintained or may decrease.

As described above, a reference of the dehumidification amount, which determines the specification of the second heater 842, may be set as the dehumidification amount dehumidified in the evaporator 341 when a specific course is performed.

For example, the clothing treatment apparatus may be provided to perform a drying course in addition to the refresh course described above.

In detail, the refresh course corresponds to a course that removes wrinkles, deodorizes, sterilizes, and dries laundry in a process of supplying steam to the laundry and drying the laundry again.

When the refresh course is performed, the controller may perform the control method shown in FIG. 21.

The controller may stop driving of the compressor in a section of an injection operation of fully injecting steam into the inner case, a standby operation of not supplying steam and hot air into the inner case, and a cooling operation of driving only a blowing fan, in a refresh course.

An object of the refresh course is to treat non-wet laundry or laundry with a moisture content of 50 percent or less, and thus the controller may prevent thermal damage to the laundry by providing a section in which supply of the steam or hot air is stopped.

However, the drying course corresponds to a laundry treating course of a laundry with a moisture content of more than 50 %.

A main object of the drying course is to dry the laundry, and thus once hot air begins to be supplied to the laundry, the supply of hot air needs to continue until drying of the laundry is completed. This is because when supply of hot air is stopped, drying delay occurs and additional energy is required to supply hot air again.

Accordingly, when the drying course is performed, the controller drives the compressor 342 to generate hot air, and the controller does not terminate and maintains driving of the compressor 342 until the drying course ends or until drying of the laundry is completed.

Therefore, unlike the refresh course, the standby operation and the cooling operation may be omitted from the drying course.

The clothing treatment apparatus may include the steam preparation operation B1 and the preheating operation B2 in the drying course. Accordingly, in the drying course, the temperature inside the inner case may be quickly raised to a temperature higher than the temperature at which laundry is to be dried.

As a result, the drying course may only perform the drying operation B6, and all of the steam preparation operation B1, the preheating operation B2, and the drying operation B6 may be performed, but the steam injection operation B3, the standby operation B4, and the cooling operation B5 may be set to be omitted.

The specification of the first heater 841 or the second heater 842 may be determined based on the drying course.

For example, the specification of the second heater 842 may be determined based on the dehumidification amount dehumidified from the evaporator 842 during a time the drying course is performed.

In detail, the second heater 842 may be provided such that the amount of steam generated in the drying course is equal to or less than the dehumidification amount of the evaporator 341 in the drying course.

The second heater 842 may be provided such that the average steam generation amount per minute in the drying course is equal to or less than the average dehumidification amount per minute in the drying course.

The second heater 842 may be provided such that the maximum steam generation amount in the drying course is equal to or less than the maximum dehumidification amount of the evaporator in the drying course.

Alternatively, the average steam generation amount of the second heater 842 in the drying course may be set to be less than the average dehumidification amount of the evaporator 341.

The drying course is performed until drying of laundry is completed, and thus a time in which the drying course is performed may be increased in the case of a large amount of laundry. In this case, the dehumidification amount dehumidified from the evaporator 342 increases, and thus it may be difficult to determine the specification of the second heater 842 based on the dehumidification amount. Therefore, the second heater 842 may be determined based on the dehumidification amount dehumidified by the evaporator 342 when the drying course is performed during a reference time. Accordingly, when the drying course is performed for the reference time, the steam generation amount of the second heater 842 may be set to be equal to or less than the dehumidification amount dehumidified by the evaporator 342.

For example, the reference time may be set to about 40 minutes. For example, the reference time may be set to 45 minutes.

The dehumidification amount of the evaporator 342 may be set assuming that sufficient moisture to be dehumidified is present inside the inner case 200 during the reference time.

The steam generation amount during the reference time in the drying course of the second heater 842 may be set to be less than the dehumidification amount during the reference time in the drying course of the evaporator 341.

When determining the specification of the second heater 842 based on the dehumidification amount, a method or reference for measuring the dehumidification amount needs to be clear.

The dehumidification amount may be defined as the amount of water condensed in the evaporator 341 when the compressor 342 is driven.

For example, the dehumidification amount may be measured as the amount of water condensed in the evaporator 341 when the compressor 342 is driven during the drying course.

The dehumidification amount may be defined based on an amount by which the weight of located laundry is reduced during a specific time or a time in which a certain course is performed.

For example, the dehumidification amount of the evaporator 341 in the drying course may be measured by the amount by which the weight of the laundry is reduced during the drying course.

The weight of the laundry may be detected by the controller 700 through changes in a driving frequency, driving RPM, or electrical energy of the moving hanger 1000.

The dehumidification amount may be measured as the amount of water reduced in the water supply tank 30 during a specific time or a certain course in which a certain course is performed.

The dehumidification amount may be measured as the increased amount of water condensed in the evaporator 341 and collected in the water collector or a drain tank 40 during a specific time or a time in which a certain course is performed.

The specifications of the first heater 841 and the second heater 842 may be determined within a certain range based on the dehumidification amount of the evaporator in any refresh course other than the drying course performed by the controller 700. The refresh courses may generally be divided into a basic course and an intensive course.

The basic course may include the standard course or a boiling course, and the intensive course may correspond to a course that treats laundry more quickly than the basic course.

The basic course may correspond to a course in which the driving rpm of the compressor 342 is a basic rpm, such as a standard course, and the intensive course may correspond to a course in which the driving rpm of the compressor 342 is stronger rpm that is higher than the basic rpm.

The basic course refers to all courses in which the driving rpm of the compressor 342 is the basic rpm, and the basic course may be classified in detail depending on a time when the compressor 342 is driven, which of the first heater 841 and the second heater 842 is driven, the amount of steam supplied inside the inner case 200, a steam injection time, and the like.

The intensive course may be defined as a course in which the average rpm of the compressor 342 is set higher than the average rpm of the compressor 342 driven in the basic course.

The intensive course may be defined as a course in which the maximum rpm of the compressor 342 is set higher than the maximum rpm of the compressor 342 driven in the basic course.

The intensive course may be defined as a course in which a duration of the compressor 342 is set higher than a duration of the compressor 342 driven in the basic course.

The intensive course may be defined as a course in which the compressor 342 is driven with a higher output than an output for driving the compressor 342 in the basic course.

For example, the standard course may correspond to a course in which either the first heater 841 or the second heater 842 is to be simultaneously driven while the compressor 342 is driven at the basic rpm, and the boiling course may correspond to a course in which the second heater 842 is simultaneously driven while the compressor 842 is driven at the basic rpm.

In the boiling course, the second heater 842 may be intermittently and repeatedly driven.

Accordingly, the boiling course is intended to maintain the temperature inside the inner case 200 at a sterilization temperature for a long time, prevent excessive moisture from being supplied inside the inner case 200, and prevent the compressor 342 from being overloaded.

The intensive course refers to all courses in which the driving rpm of the compressor 342 is the intensive course, and the intensive course may also be classified in detail depending on a time when the compressor 342 is driven, which of the first heater 841 and the second heater 842 is driven, the amount of steam supplied inside the inner case 200, a steam injection time, and the like.

The basic course and the intensive course may each be designated as one specific course.

As a result, when the basic course is a course in which normal hot air is supplied to the inside of the inner case, and the intensive course is a course in which hot air with a higher temperature than the basic course is supplied inside the inner case to faster treat laundry than in the basic course.

The driving rpm of the compressor 342 is the same for all courses corresponding to the basic course, and thus the dehumidification amount dehumidified in the evaporator 341 may be similar.

The driving rpm of the compressor 342 is the same for all courses corresponding to the intensive course, and thus the dehumidification amount dehumidified in the evaporator 341 may be similar. The dehumidification amount of the evaporator 341 in the intensive course may be greater than the dehumidification amount of the evaporator 341 in the basic course.

At least one of the first heater 841 and the second heater 842 may be provided to have a specification such that the amount of steam generated in the basic course corresponds to 80 to 120 % of the dehumidification amount of the evaporator.

For example, the amount of steam generated by the second heater 842 may be determined to be 80 to 120% of the dehumidification amount of the evaporator 841 in the basic course.

For example, the second heater 842 may be determined to have a steam injection amount in the range of 80 to 120 % based on the dehumidification amount of the evaporator 841 in the preheating section B2 during a refresh course.

The preheating section B2 is a section in which the compressor 342 and the second heater 842 are to be simultaneously driven, and thus the dehumidification amount and the steam injection amount generated from the second heater 842 may be intuitively compared.

For example, this may correspond to the case in which the second heater 842 has a specification of 450 to 700 W, and in detail, may correspond to a specification of 600 W.

At least one of the first heater 841 and the second heater 842 may be provided to have a specification such that the amount of steam generated in the intensive course corresponds to 110 to 150 % of the dehumidification amount of the evaporator.

For example, the amount of steam generated by the first heater 841 may be determined to be 110 to 150 % of the dehumidification amount of the evaporator 841 in the intensive course. For example, the first heater 841 may be determined to have a specification of 800 to1000 W, such as 800 W or 950 W.

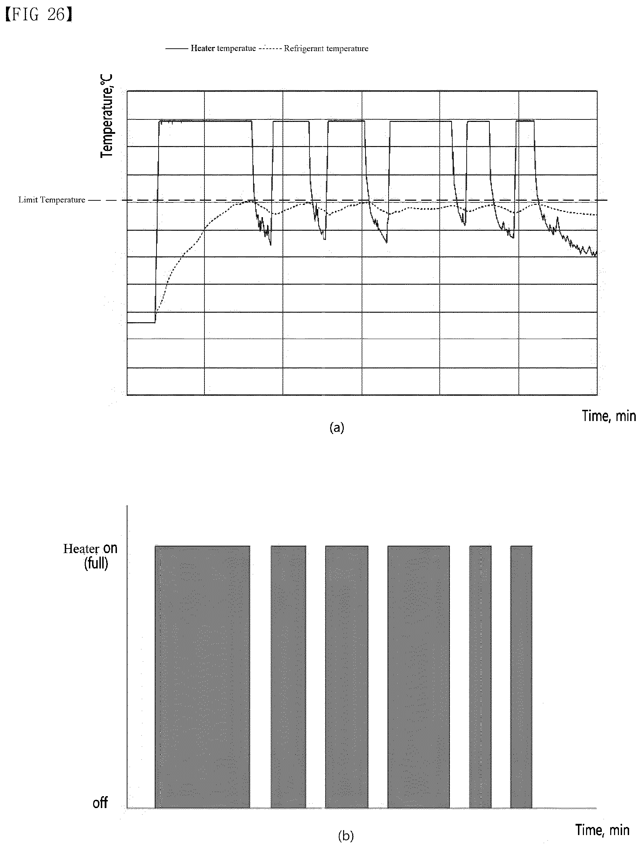

FIG. 26 shows an example of an optimal driving method of the steam heater.

In the clothing treatment apparatus, either the first heater 841 or the second heater 842 and the compressor 342 may be driven simultaneously.

Referring to FIG. 26(a), the controller 700 may intermittently and repeatedly any one of the first heater 841 and the second heater 842 when the compressor 342 is driven in at least some sections while the course is performed.

The controller may intermittently and repeatedly drive either the first heater 841 or the second heater 842 during an entire driving section of the compressor 342, but may intermittently and repeatedly drive either the first heater 841 or the second heater 842 only in a portion of a section in which the compressor 342 is driven.

When either the first heater 841 or the second heater 842 is driven, steam is supplied to the inner case 200, the internal temperature increases, and the temperature of air flowing into the evaporator 341 also increases. Accordingly, the temperature of a refrigerant flowing into the compressor 342 also increases, and the temperature of the refrigerant discharged from the compressor 342 also increases.

As a result, the pressure inside the compressor 342 may also increase, causing excessive load to be applied to the compressor 342.

When the refrigerant is R290 or CO2, which has a larger heat capacity than R-139, the reliability of the compressor 342 may decrease when the temperature of the refrigerant flowing into or discharged from the compressor 342 increases.

In summary, the controller 700 may continuously drive either the first heater 841 or the second heater 842 when the temperature of the refrigerant discharged from the compressor 342 is low. However, when the temperature of the refrigerant discharged from the compressor 342 increases rapidly or the temperature of the refrigerant discharged from the compressor 342 reaches a limit temperature that may adversely affects the reliability of the compressor 342, the controller 700 may intermittently drive the first heater 841 or the second heater 842. That is, the controller 700 may repeatedly turn ON/OFF either the first heater 841 or the second heater 842 while driving the compressor 342.

That is, the controller 700 may intermittently and alternately drive the first heater 841 or the second heater 842 when the temperature of the refrigerant flowing into or discharged from the compressor 342 reaches the limit temperature while the first heater 841 or the second heater 842 and the compressor 342 are simultaneously driven.

As a result, the compressor 342 may be driven continuously without the temperature of the refrigerant discharged from the compressor 342 exceeding the limit temperature. Accordingly, the compressor 342 may ensure reliability throughout the entire course and prevent course delay.

Driving the first heater 841 and the second heater 842 simultaneously with the compressor 342 is to quickly increase one or more of the temperature or humidity inside the inner case 200. Therefore, referring to FIG. 26(b), when the first heater 841 or the second heater 842 is driven intermittently, a driving time may be set to be longer than a time when driving is stopped.

The controller 700 may control driving of the steam heater 840 according to the temperature inside the inner case 200.

In detail, the controller 700 may intermittently and alternately drive the first heater 841 or the second heater 842 when the temperature inside the inner case 200 reaches the target temperature while the first heater 841 or the second heater 842 and the compressor 842 are simultaneously driven.

As a result, thermal damage to laundry may be prevented by preventing the temperature inside the inner case 200 from overheating above the target temperature, and even though the temperature is before the laundry is thermally damaged, the target temperature may be maintained for more than the sterilization time, thereby eradicating bacteria and mites present on the laundry.

The sterilization time may be set to 10 minutes or more.

The temperature inside the inner case 200 is a temperature corresponding to the upper side of the inner case 200 in which the moving hanger 1000 is mounted or is a temperature of air flowing into a circulation duct 310 from the inner case 200.

The controller 700 may simultaneously drive the moving hanger 1000 when driving the compressor 342, the first heater 841, or the second heater 842.

The controller 700 may change a driving frequency of the moving hanger 1000 and drive the moving hanger 1000 according to the amount of steam supplied inside the inner case 200.

For example, the controller 700 may drive the moving hanger 1000 faster when the first heater 841 is driven than when the second heater 842 is driven. This allows the laundry to be more evenly exposed to steam.

## Claims

1. A clothing treatment apparatus comprising:
a cabinet (100);
an inner case (200) provided inside the cabinet (100) to accommodate laundry;
a heat supply portion (340) including a circulation duct (320) configured to circulate air discharged from the inner case (200), an evaporator (341) installed in the circulation duct (320) to dehumidify the air, a condenser (343) configured to heat the dehumidified air, and a compressor (342) configured to compress and supply a refrigerant heating the air in the condenser (343),
a steam case (810) configured to store water generating steam supplied to the inner case (200); and
a steam supply portion (800) including a steam heater (840) accommodated in the steam case (810) and configured to heat the water to generate the steam,
**characterized in that**:
the clothes treatment apparatus further comprises a controller (700) configured to perform a refresh course in which the compressor (342) and the steam heater (840) are driven to treat laundry but a section is set in which driving of the compressor (342) is stopped, and a drying course in which the compressor (342), optionally with the steam heater (840), is driven to treat laundry but driving of the compressor (342) is maintained when driving of the compressor (342) starts until the course is terminated,
the steam heater (840) includes a first heater (841) configured to heat the water to generate steam, and a second heater (842) provided independently from the first heater (841) and configured to heat the water to generate steam,
the refresh course includes a preheating operation of simultaneously driving the first heater (841) or the second heater (842) and the compressor (342),
the first heater (841) or the second heater (842) is provided to have a specification in which an amount of steam generated in the preheating operation corresponds to 80 to 120 % of a dehumidification amount of the evaporator (341), and
the dehumidification amount is measured based on an amount of water condensed by the evaporator.

2. The clothing treatment apparatus of claim 1, wherein the first heater (841) or the second heater (842) is provided to have a specification in which an amount of steam generated in the drying course is equal to or less than the dehumidification amount of the evaporator (341) in the drying course.

3. The clothing treatment apparatus of claim 2, wherein the first heater (841) or the second heater (842) is provided to have a specification in which an amount of steam generated during a reference time in the drying course is equal to or less than the dehumidification amount of the evaporator (341) during a reference time in the drying course.

4. The clothing treatment apparatus of claim 1, wherein the refresh course includes a basic course in which the compressor (342) and the steam heater (840) are driven, and an intensive course in which the compressor (342) is driven at driving rpm greater than the basic course, and
the first heater (841) is provided to have a specification in which an amount of steam generated in the intensive course corresponds to 90 to 150 % of the dehumidification amount of the evaporator (341).

5. The clothing treatment apparatus of claim 1, wherein an amount of steam generated by the second heater (842) is set to be less than the first heater (841).

6. The clothing treatment apparatus of claim 1, wherein the first heater (841) is configured to generate steam greater than a dehumidification amount of the evaporator (341), and
the second heater (842) is configured to generate steam equal to or less than the dehumidification amount of the evaporator (341).

7. The clothing treatment apparatus of claim 6, wherein the first heater (841) is configured to have an average steam generation amount per minute, greater than an average dehumidification amount per minute of the evaporator (341), and
the second heater (842) is configured to have an average steam generation amount per minute, equal to or less than the average dehumidification amount per minute of the evaporator (341).

8. The clothing treatment apparatus of claim 6, wherein the first heater (841) is configured to have a maximum steam generation amount greater than a maximum dehumidification amount of the evaporator (341), and
the second heater (842) is configured to have the maximum steam generation amount equal to or less than the maximum dehumidification amount of the evaporator (341).

9. The clothing treatment apparatus of claim 6, wherein, in case that the compressor (342) and the first heater (841) are driven simultaneously for more than a certain time, an internal humidity of the inner case (200) is set to increase.

10. The clothing treatment apparatus of claim 6, wherein, in case that the compressor (342) and the second heater (842) are driven simultaneously for more than a certain time, an internal humidity of the inner case (200) is set to be maintained or decrease.

11. The clothing treatment apparatus of claim 6, wherein, in case that the compressor (342), the first heater (841), and the second heater (842) are driven simultaneously for more than a certain time, an inner humidity of the inner case (200) is to increase.

12. The clothing treatment apparatus of claim 11, wherein an average steam generation amount of the second heater (842) is set to be less than an average dehumidification amount of the evaporator (341).

## Patentansprüche

1. Vorrichtung zur Kleidungsbehandlung, umfassend:
einen Schrank (100);
ein Innengehäuse (200), das innerhalb des Schranks (100) bereitgestellt ist, um Wäsche aufzunehmen;
einen Wärmezufuhrabschnitt (340), der einen Zirkulationskanal (320), der dazu konfiguriert ist, aus dem Innengehäuse (200) abgeführte Luft zu zirkulieren, einen Verdampfer (341), der in dem Zirkulationskanal (320) installiert ist, um die Luft zu entfeuchten, einen Kondensator (343), der dazu konfiguriert ist, die entfeuchtete Luft zu erwärmen, und einen Kompressor (342), der dazu konfiguriert ist, ein Kältemittel, das die Luft in dem Kondensator (343) erwärmt, zu komprimieren und zuzuführen, umfasst,
ein Dampfgehäuse (810), das dazu konfiguriert ist, Wasser zu speichern, das Dampf erzeugt, der dem Innengehäuse (200) zugeführt wird; und
einen Dampfzufuhrabschnitt (800), der einen Dampferhitzer (840) umfasst, der in dem Dampfgehäuse (810) aufgenommen ist und dazu konfiguriert ist, das Wasser zu erhitzen, um den Dampf zu erzeugen,
**dadurch gekennzeichnet, dass**:
die Vorrichtung zur Kleidungsbehandlung ferner eine Steuerung (700) umfasst, die dazu konfiguriert ist, einen Auffrischungsgang durchzuführen, in dem der Kompressor (342) und der Dampferhitzer (840) angetrieben werden, um Wäsche zu behandeln, wobei jedoch ein Abschnitt festgelegt ist, in dem der Antrieb des Kompressors (342) gestoppt wird, und einen Trocknungsgang, in dem der Kompressor (342), optional mit dem Dampferhitzer (840), angetrieben wird, um Wäsche zu behandeln, wobei jedoch der Antrieb des Kompressors (342) aufrechterhalten wird, wenn der Antrieb des Kompressors (342) startet, bis der Gang beendet ist,
der Dampferhitzer (840) einen ersten Erhitzer (841), der dazu konfiguriert ist, das Wasser zu erhitzen, um Dampf zu erzeugen, und einen zweiten Erhitzer (842), der unabhängig von dem ersten Erhitzer (841) bereitgestellt ist und dazu konfiguriert ist, das Wasser zu erhitzen, um Dampf zu erzeugen, umfasst,
der Auffrischungsgang einen Vorheizvorgang umfasst, bei dem der erste Erhitzer (841) oder der zweite Erhitzer (842) und der Kompressor (342) gleichzeitig angetrieben werden,
der erste Erhitzer (841) oder der zweite Erhitzer (842) so bereitgestellt ist, dass er eine Spezifikation aufweist, bei der eine in dem Vorheizvorgang erzeugte Dampfmenge 80 bis 120 % einer Entfeuchtungsmenge des Verdampfers (341) entspricht, und
die Entfeuchtungsmenge auf Grundlage einer Menge an durch den Verdampfer kondensiertem Wasser gemessen wird.

2. Vorrichtung zur Kleidungsbehandlung nach Anspruch 1, wobei der erste Erhitzer (841) oder der zweite Erhitzer (842) so bereitgestellt ist, dass er eine Spezifikation aufweist, bei der eine in dem Trocknungsgang erzeugte Dampfmenge gleich der Entfeuchtungsmenge des Verdampfers (341) in dem Trocknungsgang oder geringer als diese ist.

3. Vorrichtung zur Kleidungsbehandlung nach Anspruch 2, wobei der erste Erhitzer (841) oder der zweite Erhitzer (842) so bereitgestellt ist, dass er eine Spezifikation aufweist, bei der eine während einer Referenzzeit in dem Trocknungsgang erzeugte Dampfmenge gleich der Entfeuchtungsmenge des Verdampfers (341) während einer Referenzzeit in dem Trocknungsgang oder geringer als diese ist.

4. Vorrichtung zur Kleidungsbehandlung nach Anspruch 1, wobei der Auffrischungsgang einen Grundgang, in dem der Kompressor (342) und der Dampferhitzer (840) angetrieben werden, und einen Intensivgang, in dem der Kompressor (342) mit einer Antriebsdrehzahl angetrieben wird, die größer ist als in dem Grundgang, umfasst, und
der erste Erhitzer (841) so bereitgestellt ist, dass er eine Spezifikation aufweist, bei der eine in dem Intensivgang erzeugte Dampfmenge 90 bis 150 % der Entfeuchtungsmenge des Verdampfers (341) entspricht.

5. Vorrichtung zur Kleidungsbehandlung nach Anspruch 1, wobei eine durch den zweiten Erhitzer (842) erzeugte Dampfmenge so festgelegt ist, dass sie geringer ist als die des ersten Erhitzers (841).

6. Vorrichtung zur Kleidungsbehandlung nach Anspruch 1, wobei der erste Erhitzer (841) dazu konfiguriert ist, Dampf in einer Menge zu erzeugen, die größer ist als eine Entfeuchtungsmenge des Verdampfers (341), und
der zweite Erhitzer (842) dazu konfiguriert ist, Dampf in einer Menge zu erzeugen, die gleich der Entfeuchtungsmenge des Verdampfers (341) oder geringer als diese ist.

7. Vorrichtung zur Kleidungsbehandlung nach Anspruch 6, wobei der erste Erhitzer (841) dazu konfiguriert ist, eine durchschnittliche Dampferzeugungsmenge pro Minute aufzuweisen, die größer ist als eine durchschnittliche Entfeuchtungsmenge pro Minute des Verdampfers (341), und
der zweite Erhitzer (842) dazu konfiguriert ist, eine durchschnittliche Dampferzeugungsmenge pro Minute aufzuweisen, die gleich der durchschnittlichen Entfeuchtungsmenge pro Minute des Verdampfers (341) oder geringer als diese ist.

8. Vorrichtung zur Kleidungsbehandlung nach Anspruch 6, wobei der erste Erhitzer (841) dazu konfiguriert ist, eine maximale Dampferzeugungsmenge aufzuweisen, die größer ist als eine maximale Entfeuchtungsmenge des Verdampfers (341), und
der zweite Erhitzer (842) dazu konfiguriert ist, die maximale Dampferzeugungsmenge aufzuweisen, die gleich der maximalen Entfeuchtungsmenge des Verdampfers (341) oder geringer als diese ist.

9. Vorrichtung zur Kleidungsbehandlung nach Anspruch 6, wobei, falls der Kompressor (342) und der erste Erhitzer (841) länger als eine bestimmte Zeit gleichzeitig angetrieben werden, eine innere Feuchtigkeit des Innengehäuses (200) so festgelegt ist, dass sie zunimmt.

10. Vorrichtung zur Kleidungsbehandlung nach Anspruch 6, wobei, falls der Kompressor (342) und der zweite Erhitzer (842) länger als eine bestimmte Zeit gleichzeitig angetrieben werden, eine innere Feuchtigkeit des Innengehäuses (200) so festgelegt ist, dass sie aufrechterhalten wird oder abnimmt.

11. Vorrichtung zur Kleidungsbehandlung nach Anspruch 6, wobei, falls der Kompressor (342), der erste Erhitzer (841) und der zweite Erhitzer (842) länger als eine bestimmte Zeit gleichzeitig angetrieben werden, eine innere Feuchtigkeit des Innengehäuses (200) zunehmen soll.

12. Vorrichtung zur Kleidungsbehandlung nach Anspruch 11, wobei eine durchschnittliche Dampferzeugungsmenge des zweiten Erhitzers (842) so festgelegt ist, dass sie geringer ist als eine durchschnittliche Entfeuchtungsmenge des Verdampfers (341).

## Revendications

1. Appareil de traitement de vêtements, comprenant :
une armoire (100) ;
un boîtier intérieur (200) prévu à l'intérieur de l'armoire (100) pour recevoir du linge ;
une portion d'apport de chaleur (340) comprenant un conduit de circulation (320) configuré pour faire circuler de l'air évacué du boîtier intérieur (200), un évaporateur (341) installé dans le conduit de circulation (320) pour déshumidifier l'air, un condenseur (343) configuré pour chauffer l'air déshumidifié, et un compresseur (342) configuré pour comprimer et fournir un réfrigérant chauffant l'air dans le condenseur (343),
un boîtier de vapeur (810) configuré pour stocker de l'eau générant de la vapeur fournie au boîtier intérieur (200) ; et
une portion d'alimentation en vapeur (800) comprenant un dispositif de chauffage de vapeur (840) logé dans le boîtier de vapeur (810) et configuré pour chauffer l'eau afin de générer la vapeur,
**caractérisé en ce que** :
l'appareil de traitement de vêtements comprend en outre un dispositif de commande (700) configuré pour exécuter un cycle de rafraîchissement dans lequel le compresseur (342) et le dispositif de chauffage de vapeur (840) sont entraînés pour traiter du linge, mais une section est définie dans laquelle l'entraînement du compresseur (342) est arrêté, et un cycle de séchage dans lequel le compresseur (342), éventuellement avec le dispositif de chauffage de vapeur (840), est entraîné pour traiter du linge, mais l'entraînement du compresseur (342) est maintenu lorsque l'entraînement du compresseur (342) démarre jusqu'à ce que le cycle soit terminé,
le dispositif de chauffage de vapeur (840) comprend un premier dispositif de chauffage (841) configuré pour chauffer l'eau afin de générer de la vapeur, et un second dispositif de chauffage (842) prévu indépendamment du premier dispositif de chauffage (841) et configuré pour chauffer l'eau afin de générer de la vapeur,
le cycle de rafraîchissement comprend une opération de préchauffage consistant à entraîner simultanément le premier dispositif de chauffage (841) ou le second dispositif de chauffage (842) et le compresseur (342),
le premier dispositif de chauffage (841) ou le second dispositif de chauffage (842) est prévu de manière à présenter une spécification selon laquelle une quantité de vapeur générée dans l'opération de préchauffage correspond à 80 à 120 % d'une quantité de déshumidification de l'évaporateur (341), et
la quantité de déshumidification est mesurée sur la base d'une quantité d'eau condensée par l'évaporateur.

2. Appareil de traitement de vêtements selon la revendication 1, dans lequel le premier dispositif de chauffage (841) ou le second dispositif de chauffage (842) est prévu de manière à présenter une spécification selon laquelle une quantité de vapeur générée dans le cycle de séchage est égale ou inférieure à la quantité de déshumidification de l'évaporateur (341) dans le cycle de séchage.

3. Appareil de traitement de vêtements selon la revendication 2, dans lequel le premier dispositif de chauffage (841) ou le second dispositif de chauffage (842) est prévu de manière à présenter une spécification selon laquelle une quantité de vapeur générée pendant un temps de référence dans le cycle de séchage est égale ou inférieure à la quantité de déshumidification de l'évaporateur (341) pendant un temps de référence dans le cycle de séchage.

4. Appareil de traitement de vêtements selon la revendication 1, dans lequel le cycle de rafraîchissement comprend un cycle de base dans lequel le compresseur (342) et le dispositif de chauffage de vapeur (840) sont entraînés, et un cycle intensif dans lequel le compresseur (342) est entraîné à un régime d'entraînement supérieur à celui du cycle de base, et
le premier dispositif de chauffage (841) est prévu de manière à présenter une spécification selon laquelle une quantité de vapeur générée dans le cycle intensif correspond à 90 à 150 % de la quantité de déshumidification de l'évaporateur (341).

5. Appareil de traitement de vêtements selon la revendication 1, dans lequel une quantité de vapeur générée par le second dispositif de chauffage (842) est définie comme étant inférieure à celle du premier dispositif de chauffage (841).

6. Appareil de traitement de vêtements selon la revendication 1, dans lequel le premier dispositif de chauffage (841) est configuré pour générer une quantité de vapeur supérieure à une quantité de déshumidification de l'évaporateur (341), et
le second dispositif de chauffage (842) est configuré pour générer une quantité de vapeur égale ou inférieure à la quantité de déshumidification de l'évaporateur (341).

7. Appareil de traitement de vêtements selon la revendication 6, dans lequel le premier dispositif de chauffage (841) est configuré pour présenter une quantité moyenne de génération de vapeur par minute supérieure à une quantité moyenne de déshumidification par minute de l'évaporateur (341), et
le second dispositif de chauffage (842) est configuré pour présenter une quantité moyenne de génération de vapeur par minute égale ou inférieure à la quantité moyenne de déshumidification par minute de l'évaporateur (341).

8. Appareil de traitement de vêtements selon la revendication 6, dans lequel le premier dispositif de chauffage (841) est configuré pour présenter une quantité maximale de génération de vapeur supérieure à une quantité maximale de déshumidification de l'évaporateur (341), et
le second dispositif de chauffage (842) est configuré pour présenter la quantité maximale de génération de vapeur égale ou inférieure à la quantité maximale de déshumidification de l'évaporateur (341).

9. Appareil de traitement de vêtements selon la revendication 6, dans lequel, dans le cas où le compresseur (342) et le premier dispositif de chauffage (841) sont entraînés simultanément pendant plus d'un certain temps, une humidité interne du boîtier intérieur (200) est définie pour augmenter.

10. Appareil de traitement de vêtements selon la revendication 6, dans lequel, dans le cas où le compresseur (342) et le second dispositif de chauffage (842) sont entraînés simultanément pendant plus d'un certain temps, une humidité interne du boîtier intérieur (200) est définie pour être maintenue ou diminuer.

11. Appareil de traitement de vêtements selon la revendication 6, dans lequel, dans le cas où le compresseur (342), le premier dispositif de chauffage (841) et le second dispositif de chauffage (842) sont entraînés simultanément pendant plus d'un certain temps, une humidité intérieure du boîtier intérieur (200) doit augmenter.

12. Appareil de traitement de vêtements selon la revendication 11, dans lequel une quantité moyenne de génération de vapeur du second dispositif de chauffage (842) est définie comme étant inférieure à une quantité moyenne de déshumidification de l'évaporateur (341).
